# FASCICULE DE BREVET EUROPEEN

(11) **EP 3 908 248 B1**
(45) Date de publication et mention de la délivrance du brevet: **01.03.2023**
(21) Numéro de dépôt: 20705416.4
(22) Date de dépôt: 10.01.2020
(51) Int. Cl.: A61K 8/31, A61Q 19/00, A61K 8/81, A61K 8/06

(54) **COMPOSITION EPAISSISSANTE BIOSOURCEE COMPRENANT UN POLY(FARNÉSÈNE)**
BIOBASIERTE VERDICKUNGSZUSAMMENSETZUNG MIT EINEM (POLY)FARNESEN
BIOBASED THICKENING COMPOSITION COMPRISING A POLY(FARNESENE)

(30) Priorité: 10.01.2019 FR 1900252
(43) Date de publication de la demande: 17.11.2021
(73) Titulaire: TotalEnergies OneTech, 92400 Courbevoie (FR)
(72) Inventeur: SWOBODA, Benjamin, 78630 Orgeval (FR)
(74) Mandataire: August Debouzy
(86) Numéro de dépôt international: PCT/FR2020/050031
(87) Numéro de publication internationale: WO 2020/144440

(56) Documents cités:
- WO-A1-2018/029143
- US-A1- 2012 010 370
- US-A1- 2013 251 846
- US-A1- 2015 051 332

## Description

### DOMAINE TECHNIQUE DE L'INVENTION

L'invention concerne une composition épaississante comprenant au moins une huile d'origine biologique et un polymère polyfarnesène.

L'invention concerne également une composition cosmétique comprenant ladite composition épaississante et son utilisation.

La présente invention concerne aussi l'utilisation de ladite composition épaississante pour la formulation de produits cosmétiques.

### ETAT DE LA TECHNIQUE

Les produits cosmétiques existent sous différentes formes. Ils peuvent être soit sous forme d'émulsions (un mélange entre une phase aqueuse et une phase grasse stabilisée par un émulsionnant), soit entièrement aqueux, soit entièrement anhydres (uniquement une phase grasse). Pour texturer ou augmenter la viscosité de la phase grasse, soit dans une émulsion soit dans un produit anhydre, l'homme du métier utilisera un épaississant de phase grasse qui peut être un gel, c'est-à-dire un mélange entre une huile apolaire et un gélifiant. Ces gels sont particulièrement utilisés pour la formulation de gloss, d'hydratant pour les lèvres, de crème pour la peau ou de produit solaire. Mais on les retrouve également dans les produits capillaires, les crèmes dépilatoires et les déodorants.

Ainsi, un gel apolaire est un mélange d'une huile apolaire et d'un gélifiant. Les huiles apolaires peuvent être des huiles minérales, des huiles végétales, des huiles silicones ou des isoparaffines. Le gélifiant est le plus souvent polymérique tel que le copolymère éthylène/propylène styrène ou le copolymère butylène/éthylène/styrène ou les copolymères polyisobutène, polydécène ou isoprène (ou encore SEBS, SBS, SIS, TPE,...). Ces copolymères sont typiquement vendus par les sociétés Kraton ou Kuraray.

Les gels les plus répandus sont les gels à base d'huile minérale. La compatibilité entre ces huiles et les copolymères est très bonne, en effet la structure apolaire des copolymères est très compatible avec ces huiles. Les inconvénients majeurs de gel à base d'huile minérale sont : l'origine fossile de l'huile et la texture filante du produit.

Le document WO 2018/172228 décrit une composition gélifiée comprenant une huile hydrocarbonée d'origine biologique et un polymère gélifiant d'origine fossile. L'huile hydrocarbonée et le polymère gélifiant sont mélangés à des températures supérieures à 40°C pour permettre l'obtention de la composition gélifiée.

Les documents US 9,334,394 et US 2015/0051332 décrivent des compositions de caoutchouc comprenant un polymère de type poly(farnesène). Ces compositions ne sont pas adaptées à une utilisation pour une application topique sur la peau, les ongles ou phanères.

Le document US 2013/0251846 décrit des compositions de chewing-gum destinées pour une administration orale. Ce document ne divulgue pas des compositions adaptées pour une application topique sur la peau, les ongles ou phanères. Ce document divulgue des compositions comprenant un polymère poly(farnesène) présentant une masse moléculaire moyenne en poids supérieure à 450000 g/mol.

Il subsiste donc le besoin de disposer d'une composition épaississante pouvant être obtenue par mélange à température ambiante, typiquement une température inférieure à 40°C, plus particulièrement inférieure à 30°C, et présentant de bonnes propriétés sensorielles et de brillance.

La demanderesse a trouvé de manière surprenante que ce besoin peut être satisfait par une nouvelle composition épaississante avec une phase grasse d'origine biologique et un polymère biosourcé de type poly(farnesène).

La présente invention a également pour objectif de fournir une composition épaississante à base de matières premières d'origine biologique, en particulier lorsque la phase grasse est d'origine biologique.

La présente invention a également pour objectif de proposer une composition épaississante stable aux propriétés adaptées à son utilisation.

### RESUME DE L'INVENTION

Ces objectifs sont atteints grâce à une nouvelle composition épaississante.

L'invention concerne une composition épaississante comprenant :
- Au moins une huile d'origine biologique, et
- Au moins un polymère poly(farnesène) ayant une masse molaire moyenne en nombre allant de 10000 à 120000 g/mol.

Selon un mode de réalisation, l'huile d'origine biologique est choisie parmi les huiles hydrocarbonées, les huiles végétales ou animales sous forme d'acides, d'esters ou d'amides, et leurs mélanges.

Selon un mode de réalisation, l'huile d'origine biologique est une huile hydrocarbonée, de préférence comprenant une teneur en poids d'isoparaffines allant de 90 à 100%, une teneur en poids de paraffines normales allant de 0 à 10% et une teneur en carbone d'origine biologique de préférence supérieure ou égale à 90 % par rapport au poids total de l'huile hydrocarbonée.

De préférence, l'huile hydrocarbonée est choisie parmi les isoparaffines non-cycliques comprenant de 14 à 18 atomes de carbone.

De préférence, l'huile hydrocarbonée comprend :
- une teneur en poids d'isoparaffines allant de 95 à 100% et préférentiellement de 98% à 100% par rapport au poids total de l'huile hydrocarbonée ; et/ou
- une teneur en carbone d'origine biologique supérieure ou égale à 95%, de préférence supérieure ou égale à 98% et préférentiellement de 100% ; et/ou
- une teneur en poids de paraffines normales inférieure ou égale à 10, de préférence inférieure ou égale à 5% et préférentiellement inférieure ou égale à 2% par rapport au poids total de l'huile hydrocarbonée ; et/ou
- une teneur en poids de composés naphténiques inférieure ou égale à 1%, de préférence inférieure ou égale à 0,5% et préférentiellement inférieure ou égale à 100 ppm par rapport au poids total de l'huile hydrocarbonée ; et/ou
- une teneur en poids de composés aromatiques inférieure ou égale à 500 ppm, de préférence inférieure ou égale à 300 ppm, préférentiellement inférieure ou égale à 100 ppm, plus préférentiellement inférieure ou égale à 50 ppm et avantageusement inférieure ou égale à 20 ppm,
par rapport au poids total de l'huile hydrocarbonée.

De préférence, l'huile hydrocarbonée est obtenue par un procédé d'hydrogénation catalytique à une température de 80 à 180°C et à une pression de 50 à 160 bars d'une charge d'origine biologique désoxygénée et/ou isomérisée.

Selon un mode de réalisation, le polymère poly(farnesène) présente une masse molaire moyenne en nombre allant de 20000 à 110000 g/mol, de préférence de 30000 à 100000 g/mol, de préférence encore de 40000 à 90000 g/mol, plus préférentiellement de 50000 à 80000 g/mol.

Selon un mode de réalisation, le polymère poly(farnesène) présente une masse molaire moyenne en nombre allant de 10000 à 95000 g/mol, de préférence de 15000 à 90000 g/mol, de préférence encore de 20000 à 85000 g/mol, plus préférentiellement de 30000 à 80000 g/mol.

Selon un mode de réalisation, le polymère poly(farnesène) est partiellement ou totalement hydrogéné et/ou le polymère poly(farnesène) est fonctionnalisé avec des hydroxyles.

Selon un mode de réalisation, la composition épaississante comprend, par rapport au poids total de la composition épaississante :
- de 10 à 90% en poids, de préférence de 20 à 80% en poids, de préférence encore de 30 à 70% en poids, encore plus préférentiellement de 40 à 60% en poids, d'huile(s) d'origine biologique, et
- de 10 à 90% en poids, de préférence de 20 à 80% en poids, de préférence encore de 30 à 70% en poids, encore plus préférentiellement de 40 à 60% en poids, de polymère(s) poly(farnesène).

La présente invention concerne également l'utilisation de la composition épaississante selon l'invention, comme agent épaississant, agent sensoriel, agent filmogène, agent texturant, agent améliorant l'imperméabilité, agent hydratant, conditionneur, huile visqueuse ou agent ayant un effet seconde peau, dans une composition cosmétique comprenant de préférence au moins un corps gras.

La présente invention a également pour objet une composition cosmétique comprenant au moins une composition épaississante selon l'invention, de préférence en une quantité allant de 1 à 80%, préférentiellement de 5 à 70% et avantageusement de 10 à 50% en poids par rapport au poids total de la composition cosmétique.

Selon un mode de réalisation, la composition cosmétique selon l'invention comprend au moins un corps gras choisi parmi : les huiles végétales le cas échéant autres que les huiles végétales de ladite composition épaississante, les huiles hydrocarbonées le cas échéant autres que l'huile hydrocarbonée de ladite composition épaississante, les beurres végétaux, les éthers et alcools gras, les esters huileux, les alcanes et les huiles silicones et/ou au moins un additif de préférence choisi parmi les émulsionnants.

Un autre objet de la présente invention concerne l'utilisation de la composition épaississante selon l'invention, ou de la composition cosmétique selon l'invention pour une application topique, notamment comme produit de soin pour la peau ou des cheveux, comme produit de maquillage, comme produit capillaire, comme produit démaquillant, comme produit parfumé, comme produit solaire, comme produit de soin des lèvres, tel que le gloss ou les sticks hydratants pour les lèvres.

Enfin, l'invention a pour objet un procédé de traitement cosmétique de la peau, des lèvres ou des phanères comprenant au moins une étape d'application, de préférence par étalement, de la composition épaississante selon l'invention ou de la composition cosmétique selon l'invention, sur la peau, les lèvres ou les phanères.

La composition épaississante selon l'invention permet de disposer d'une composition classée non-irritante, biodégradable et non-odorante.

La composition épaississante selon l'invention est d'origine biosourcée.

La composition épaississante selon l'invention peut être obtenue par simple mélange à température ambiante d'une huile d'origine biologique et d'un polymère d'origine biologique.

La composition épaississante selon l'invention peut être utilisée comme agent sensoriel dans les compositions cosmétiques, en particulier dans les phases grasses des compositions cosmétiques.

La composition épaississante selon l'invention peut être utilisée comme agent de brillance dans des compositions cosmétiques, telles que des gloss.

La composition épaississante selon l'invention peut être utilisée comme agent filmogène, huile visqueuse, épaississant, agent texturant.

### DESCRIPTION DETAILLEE DE L'INVENTION

L'invention concerne une composition épaississante pour une application topique sur la peau, les ongles ou les phanères (incluant les lèvres, les cheveux et le cuir chevelu), ladite composition épaississante comprenant :
- De 10 à 90% en poids d'au moins une huile d'origine biologique, et
- De 10 à 90% en poids d'au moins un polymère poly(farnesène) ayant une masse molaire moyenne en nombre allant de 10000 à 120000 g/mol, de préférence de 10000 à 95000 g/mol,
par rapport au poids total de la composition épaississante.

A titre préliminaire on notera que, dans la description et les revendications suivantes, l'expression « compris entre » doit s'entendre comme incluant les bornes citées.

### Huile d'origine biologique

L'huile d'origine biologique utilisée dans la composition épaississante selon l'invention peut être choisie parmi :
- les huiles hydrocarbonées d'origine biologique,
- les huiles végétales ou animales animale choisies parmi les acides gras en C₆ à C₂₄ sous forme acide, ester ou amide, de préférence sous forme acide ou ester,
- et leur mélange.

### Huiles végétales ou animales

Selon un mode de réalisation, l'huile d'origine biologique est choisie parmi les huiles végétales ou animales animale choisies parmi les acides gras en C₆ à C₂₄ sous forme acide, ester ou amide, de préférence parmi les acides gras en C₁₂ à C₂₄ sous forme acide ou ester.

On entend par ester ou amide d'acides gras les produits définis comme suit :
- Un ester d'acide gras est le produit de réaction entre au moins un acide gras et au moins un alcool d'origine biologique, cet alcool pouvant être un mono-alcool, linéaire ou ramifié, comprenant de 1 à 6 atomes de carbones, ou un polyol, linéaire ou ramifié, comprenant de 2 à 5 groupements hydroxyles de préférence un triméthylolpropane, un érythritol, un pentaérythritol, un glycol, et/ou un glycérol. On peut ainsi obtenir des monoesters, des diesters, des triesters, des tétraesters ou des pentaesters. Sont inclus dans cette définition, les huiles végétales elles-mêmes et leurs produits de trans-estérification.
- Un amide d'acide gras est le produit de réaction entre au moins un acide gras et au moins une amine primaire, secondaire ou tertiaire ou des polyamines d'origine biologique comprenant de 2 à 6 groupements aminés.

Parmi les composés d'origine végétale, on choisira les acides, esters ou amides des huiles de tall (tall oil en anglais), de colza, de tournesol, de ricin, d'arachide, de lin, de coprah, d'olive, de palme, de coton, de mais, de suif, de saindoux, de palmiste, de soja, de courge, de pépin de raisin, d'argan, de jojoba, de sésame, de noix, de noisette, de bois de chine, de riz ainsi que les huiles de même type issues d'espèces hybrides ou génétiquement modifiées.

Parmi les composés d'origine animale, on peut citer les acides, esters et amides de graisses d'animaux marins, poissons ou mammifères marins et les graisses d'animaux terrestres telles que les graisses de cheval, de boeuf, de porc.

On préfère les acides gras d'huile de tall (ou tall oil fatty acids en anglais ou TOFA), contenant de 0,5 à 10 % en poids d'acide résinique et ses dérivés ester à base de méthanol, d'éthanol, de glycol et de glycérol.

On préfère aussi les triglycérides et autres esters d'huile de soja et de colza, y compris de leurs hybrides ou espèces génétiquement modifiées.

L'huile d'origine végétale ou animale utilisée selon l'invention peut aussi être choisie parmi les esters méthyliques d'huile végétale (EMHV).

Ainsi, selon un mode de réalisation particulier, l'huile d'origine végétale ou animale mise en oeuvre selon l'invention est choisie parmi les esters méthyliques d'huile végétale (EMHV), les acides gras d'huile de tall (ou tall oil fatty acids en anglais ou TOFA), et leurs mélanges.

L'huile d'origine biologique mise en oeuvre dans l'invention présente typiquement une teneur en biomatériau d'au moins 90%. Cette teneur est avantageusement plus élevée, en particulier supérieure ou égale à 95%, préférablement supérieure ou égale à 98 %et avantageusement égale à 100%. La détermination de la teneur en biomatériau ou en bio-carbone est donnée conformément aux normes ASTM D 6866-12, la méthode B (ASTM D 6866-06) et ASTM D 7026 (ASTM D 7026-04).

Le terme de « bio-carbone » indique que le carbone est d'origine naturelle et vient d'un biomatériau, comme indiqué ci-après. La teneur en bio-carbone et la teneur en biomatériau sont des expressions indiquant la même valeur. Un matériau d'origine renouvelable ou biomatériau est un matériau organique dans lequel le carbone est issu du CO₂ fixé récemment (sur une échelle humaine) par photosynthèse à partir de l'atmosphère. Un biomatériau (Carbone 100% d'origine naturelle) présente un rapport isotopique ¹⁴C /¹²C supérieur à 10⁻¹², typiquement environ 1,2 × 10⁻¹², tandis qu'un matériau fossile a un rapport nul. En effet, le ¹⁴C isotopique est formé dans l'atmosphère et est alors intégré par photosynthèse, selon une échelle de temps de quelques dizaines d'années au maximum. La demi-vie du ¹⁴C est 5730 années. Ainsi, les matériaux issus de la photosynthèse, à savoir les plantes d'une manière générale, ont nécessairement un contenu maximum en isotope ¹⁴C.

### Huiles hydrocarbonées

Selon un mode de réalisation préféré, l'huile d'origine biologique utilisée dans la composition épaississante selon l'invention est une huile hydrocarbonée.

De préférence, l'huile hydrocarbonée comprend des molécules présentant de 8 à 30 atomes de carbone, de préférence de 12 à 30 atomes de carbone, de préférence encore de 13 à 19 atomes de carbone, voire de 14 à 18 atomes de carbone.

La composition épaississante selon l'invention comprend de préférence une teneur d'huile hydrocarbonée allant de 10 à 90% en poids, préférentiellement de 20 à 80% en poids, plus préférentiellement de 30 à 70% en poids et avantageusement de 40 à 60% en poids par rapport au poids total de la composition.

La présence d'une quantité significative d'huile hydrocarbonée selon l'invention contribue aux qualités cosmétiques de la composition épaississante : toucher agréable, soin, brillance et protection de la peau.

L'huile hydrocarbonée de la composition épaississante selon l'invention comprend de préférence une teneur en poids de composés isoparaffiniques supérieure ou égale à 90%, préférentiellement supérieure ou égale à 95 % et avantageusement supérieure ou égale à 98% par rapport au poids total de l'huile hydrocarbonée.

Selon un mode de réalisation, les composés isoparaffiniques présents dans l'huile hydrocarbonée utilisée selon l'invention comportent de 12 à 30 atomes de carbone, de préférence de 13 à 19 atomes de carbone, de préférence encore de 14 à 18 atomes de carbone.

L'huile hydrocarbonée de la composition épaississante selon l'invention comprend de préférence une teneur en poids de paraffines normales inférieure ou égale à 10%, préférentiellement inférieure ou égale à 5 % et avantageusement inférieure ou égale à 2%.

L'huile hydrocarbonée de la composition épaississante selon l'invention comprend avantageusement une majorité d'isoparaffines et une minorité de paraffines normales. Ces isoparaffines sont avantageusement des isoparaffines non-cycliques. De préférence l'huile hydrocarbonée de la composition épaississante a un ratio massique d'isoparaffines sur paraffines normales d'au moins 12 :1, préférentiellement de 15 :1 et plus préférentiellement de 20 :1. De manière encore plus avantageuse l'huile hydrocarbonée de la composition épaississante selon l'invention ne contient pas de paraffines normales.

Selon un mode de réalisation, l'huile hydrocarbonée selon l'invention comprend de préférence une teneur en poids d'isoparaffines allant de 90 à 100% et une teneur en paraffines normales allant de 0 à 10%, préférentiellement de 95 à 100% d'isoparaffines et de 0 à 5% de paraffines normales et plus préférentiellement de 98% à 100% d'isoparaffines et de 0 à 2 % de paraffines normales.

Selon un mode de réalisation, l'huile hydrocarbonée de la composition épaississante selon l'invention comprend de préférence une teneur en poids d'isoparaffines allant de 90 à 100% et une teneur en paraffines normales allant de 0 à 10%, préférentiellement de 95 à 100% d'isoparaffines choisies parmi les alcanes comportant de 12 à 30 atomes de carbone, de préférence de 13 à 19 atomes de carbone, de préférence encore de 14 à 18 atomes de carbone.

Selon un mode de réalisation, l'huile hydrocarbonée mise en oeuvre selon l'invention comprend :
- des isoparaffines ayant 15 atomes de carbone et des isoparaffines ayant 16 atomes de carbone en une quantité combinée allant de 80 à 98% en poids, par rapport au poids total de l'huile hydrocarbonée, ou
- des isoparaffines ayant 16 atomes de carbone, des isoparaffines ayant 17 atomes de carbone et des isoparaffines ayant 18 atomes de carbone en une quantité combinée allant de 80 à 98% en poids, par rapport au poids total de l'huile hydrocarbonée, ou
- des isoparaffines ayant 17 atomes de carbone et des isoparaffines ayant 18 atomes de carbone en une quantité combinée allant de 80 à 98% en poids, par rapport au poids total de l'huile hydrocarbonée.

L'huile hydrocarbonée de la composition épaississante selon l'invention comprend de préférence une teneur en poids de composés naphténiques inférieure ou égale à 1%, préférentiellement inférieure ou égale à 0,5% et plus préférentiellement inférieure ou égale à 100 ppm.

Selon un autre mode de réalisation préféré, l'huile hydrocarbonée de la composition épaississante selon l'invention comprend une teneur en poids d'isoparaffines allant de 90 à 100%, une teneur en poids de paraffines normales allant de 0 à 10% et une teneur en poids de naphtènes inférieure ou égale à 1%. Préférentiellement l'huile hydrocarbonée comprend une teneur en poids allant de 95 à 100% d'isoparaffines, de 0 à 5% de paraffines normales et une teneur en poids de naphtènes inférieure ou égale à 0,5%. Plus préférentiellement elle comprend une teneur en poids allant de 98% à 100% d'isoparaffines, de 0 à 2 % de paraffines normales et une teneur en poids de naphtènes inférieure ou égale à 100 ppm.

L'huile hydrocarbonée mise en oeuvre dans la composition épaississante selon l'invention est avantageusement exempte de composés aromatiques. Par exempte, on entend une teneur en poids de composés aromatiques inférieure ou égale à 500 ppm, de préférence inférieure ou égale à 300 ppm, préférentiellement inférieure ou égale à 100 ppm, plus préférentiellement inférieure ou égale à 50 ppm et avantageusement inférieure ou égale à 20 ppm mesurée par exemple par spectrométrie UV.

La teneur en poids en isoparaffines, en n-paraffines, en naphtènes et/ou en aromatiques de l'huile hydrocarbonée peut être déterminée selon des méthodes bien connues de l'homme du métier. On peut citer à titre d'exemple non limitatif, une méthode par chromatographie en phase gazeuse.

Selon un autre mode de réalisation préféré, l'huile hydrocarbonée de la composition épaississante selon l'invention comprend une teneur en poids d'isoparaffines allant de 90 à 100%, une teneur en poids de paraffines normales allant de 0 à 10%, une teneur en poids de naphtènes inférieure ou égale à 1% et une teneur en poids de composés aromatiques inférieure ou égale à 500 ppm. Préférentiellement l'huile hydrocarbonée comprend une teneur en poids allant de 95 à 100% d'isoparaffines, de 0 à 5% de paraffines normales, une teneur en poids de naphtènes inférieure ou égale à 0,5% et une teneur en poids de composés aromatiques inférieure ou égale à 300 ppm, de préférence inférieure à 100 ppm, préférentiellement inférieure à 50 ppm et avantageusement inférieure à 20 ppm. Préférentiellement aussi l'huile hydrocarbonée comprend une teneur en poids allant de 95 à 100% d'isoparaffines, de 0 à 5% de paraffines normales et une teneur en poids de composés aromatiques inférieure ou égale à 100 ppm. Plus préférentiellement elle comprend une teneur en poids allant de 98% à 100% d'isoparaffines, de 0 à 2 % de paraffines normales, une teneur en poids de naphtènes inférieure ou égale à 100 ppm et une teneur en poids de composés aromatiques inférieure ou égale à 100 ppm.

L'huile hydrocarbonée mise en oeuvre dans la composition épaississante selon l'invention a également de préférence une teneur en poids de composés soufrés extrêmement basse, typiquement inférieure ou égale à 5 ppm, préférentiellement inférieure ou égale à 3 ppm et plus préférentiellement inférieure ou égale à 0,5 ppm, c'est-à-dire à un niveau trop bas pour être détectée grâce à des analyseurs de basse-teneur en soufre conventionnels.

L'huile hydrocarbonée mise en oeuvre dans la composition épaississante selon l'invention a également de préférence un point éclair supérieur ou égal à 110°C, préférentiellement supérieur ou égal à 120°C et plus préférentiellement supérieur ou égal à 140°C selon la norme EN ISO 2719. Un point éclair élevé, typiquement supérieure à 110°C permettant entre autre de pallier d'une part les problèmes de sécurité lors du stockage et du transport en évitant une inflammabilité trop sensible de l'huile hydrocarbonée.

L'huile hydrocarbonée a aussi de préférence une pression de vapeur à 20°C inférieure ou égale à 0,01 kPa.

Selon un mode de réalisation, l'huile hydrocarbonée mise en oeuvre dans la composition épaississante selon l'invention a également de préférence un point éclair supérieur ou égal à 110°C selon la norme EN ISO 2719 et une pression de vapeur à 20°C inférieure ou égale à 0,01kPa. Préférentiellement l'huile hydrocarbonée a un point éclair supérieur ou égal à 120°C et une pression de vapeur à 20°C inférieure ou égale à 0,01kPa. Et plus préférentiellement, elle a un point éclair supérieur ou égal à 130°C et une pression de vapeur à 20°C inférieure ou égale à 0,01kPa.

L'huile hydrocarbonée mise en oeuvre dans la composition épaississante selon l'invention présente des températures d'ébullition, un point éclair et une pression de vapeur permettant de pallier les problèmes d'inflammabilité, d'odeur et de volatilité.

L'huile hydrocarbonée de la composition épaississante selon l'invention a en outre de préférence une viscosité cinématique à 40°C inférieure ou égale à 5 cSt, préférentiellement inférieure ou égale à 4 cSt et plus préférentiellement inférieure ou égale à 3 cSt selon la norme EN ISO 3104.

Selon un mode particulier de réalisation, l'huile hydrocarbonée a :
- une température d'ébullition allant de 230 à 340°C, de préférence de 235 à 330°C et plus préférentiellement de 240 à 325°C selon la norme ASTM D86 ; et/ou
- une biodégradabilité à 28 jours d'au moins 60%, de préférence d'au moins 70%, préférentiellement d'au moins 75% et encore plus préférentiellement d'au moins 80% mesurée selon la norme OCDE 306 ; et/ou
- un point éclair supérieur ou égal à 110°C selon la norme EN ISO 2719.

### Procédé d'obtention de l'huile hydrocarbonée :

De telles compositions d'huiles hydrocarbonées peuvent être obtenues de la façon suivante. L'huile hydrocarbonée selon l'invention est une coupe hydrocarbonée qui est issue de la conversion de la biomasse.

Par issue de la conversion de la biomasse, on entend une coupe hydrocarbonée produite à partir de matières premières d'origine biologique.

De préférence, la coupe hydrocarbonée d'origine biologique est obtenue par un procédé comprenant des étapes d'hydrodésoxygénation (HDO) et d'isomérisation (ISO). L'étape d'hydrodésoxygénation (HDO) conduit à la décomposition des structures des esters biologiques ou des constituants triglycérides, à l'élimination des composés oxygénés, phosphorés et soufrés et à l'hydrogénation des liaisons oléfiniques. Le produit issu de la réaction d'hydrodésoxygénation est ensuite isomérisé. Une étape de fractionnement peut de préférence suivre les étapes d'hydrodésoxygénation et d'isomérisation. De manière avantageuse, les fractions d'intérêt sont ensuite soumises à des étapes d'hydrotraitement puis de distillation afin obtenir les spécifications de l'huile hydrocarbonée souhaitée selon l'invention.

Ce procédé HDO/ISO est mis en oeuvre sur une charge biologique brute, encore appelée biomasse ou matière première d'origine biologique, sélectionnée dans le groupe consistant en des huiles végétales, des graisses animales, des huiles de poisson et leur mélange. Les matières premières d'origine biologique appropriées sont par exemple l'huile de colza, l'huile de canola, l'huile de tall ou talloil, l'huile de tournesol, l'huile de soja, l'huile de chanvre, l'huile d'olive, l'huile de lin, l'huile de moutarde, l'huile de palme, l'huile d'arachide, l'huile de ricin, l'huile de noix de coco, les graisses animales telles que le suif, les graisses alimentaires recyclées, les matières premières issues du génie génétique, et les matières premières biologiques produites à partir de microorganismes tels que les algues et les bactéries. Des produits de condensation, esters ou autres dérivés obtenus à partir de matériaux biologiques bruts peuvent également servir de matières premières.

De préférence, la matière première d'origine biologique est un ester ou un dérivé triglycéride. Ce matériau est soumis tout d'abord à une étape d'hydrodésoxygénation (HDO) pour décomposer la structure des esters ou triglycérides constitutifs et éliminer les composés oxygénés, phosphorés et soufrés de manière concomitante à l'hydrogénation des liaisons oléfiniques. Cette étape d'hydrodésoxygénation (HDO) de la matière première d'origine biologique est suivie par une isomérisation du produit ainsi obtenu conduisant à la ramification de la chaîne hydrocarbonée et à une amélioration des propriétés de la paraffine à basses températures.

Durant l'étape HDO, l'hydrogène et la matière première d'origine biologique sont passés sur un lit catalytique d'hydrodésoxygénation de manière simultanée, dans le même sens ou à contre-courant. Durant l'étape HDO, la pression et la température sont comprises entre 20 et 150 bars et entre 200 et 500°C respectivement. Des catalyseurs classiques et connus d'hydrodésoxygénation sont utilisés durant cette étape. Éventuellement, la matière première d'origine biologique peut être soumise à une pré-hydrogénation sous conditions douces pour éviter les réactions secondaires des doubles liaisons avant l'étape HDO. Après l'étape d'hydrodésoxygénation, le produit issu de la réaction est soumis à une étape d'isomérisation (ISO) où l'hydrogène et le produit, et éventuellement un mélange de n-paraffines, sont passés sur des lits catalytiques d'isomérisation de manière simultanée, dans le même sens ou à contre-courant. Lors de l'étape ISO, la pression et la température sont comprises entre 20 et 150 bars et entre 200 et 500°C respectivement. Des catalyseurs classiques et connus d'isomérisation sont utilisés durant cette étape.

Des procédés secondaires additionnels peuvent également être mise en oeuvre (comme des mélanges intermédiaires, des piégeages ou autres procédés de la sorte).

Le produit issu des étapes HDO/ISO peut éventuellement être fractionné afin d'obtenir les coupes d'intérêt.

Divers procédés HDO/ISO sont décrits dans la littérature. La demande WO2014/033762 décrit un procédé comprenant une étape de pré-hydrogénation, une étape d'hydrodésoxygénation (HDO) et une étape d'isomérisation opérées à contre-courant. La demande de brevet EP1728844 décrit un procédé de production de composés hydrocarbonés à partir d'un mélange de composés d'origine végétale et animale. Ce procédé comprend une étape de prétraitement du mélange permettant d'enlever les contaminants, comme par exemple les sels de métaux alcalins, suivie d'une étape d'hydrodésoxygénation (HDO) et d'une étape d'isomérisation. La demande de brevet EP2084245 décrit un procédé de production d'un mélange hydrocarboné qui peut être utilisé comme gazole ou dans une composition de gazole par hydrodésoxygénation d'un mélange d'origine biologique contenant des esters d'acides gras éventuellement en mélange avec des acides gras libres, par exemple des huiles végétales comme l'huile de tournesol, l'huile de colza, l'huile de canola, l'huile de palme ou l'huile de tall, suivi d'une hydroisomérisation sur des catalyseurs spécifiques. La demande de brevet EP2368967 décrit un tel procédé et le produit obtenu par ce procédé. La demande WO2016/185046 décrit un procédé d'obtention d'une huile hydrocarbonée utilisée selon l'invention, dans lequel l'huile hydrocarbonée est obtenue par un procédé d'hydrogénation catalytique à une température de 80 à 180°C et à une pression de 50 à 160 bars d'une charge d'origine biologique désoxygénée et isomérisée.

Avantageusement, la matière première d'origine biologique contient moins de 15 ppm de soufre, de préférence moins de 8 ppm, préférentiellement moins de 5 ppm et plus préférentiellement moins de 1 ppm selon la norme EN ISO 20846. Idéalement la charge ne comprend pas de soufre en tant que matière première d'origine biosourcée.

Avant l'étape d'hydrotraitement, une étape de préfractionnement peut avoir lieu. Une coupe plus étroite en entrée d'unité d'hydrogénation permet d'obtenir une coupe étroite en sortie d'unité. En effet, les points d'ébullition de coupes préfractionnées sont compris entre 220 et 330 °C tandis que les coupes qui n'ont pas été préfractionnées ont typiquement des points d'ébullition comprise entre 150 et 360°C.

La charge désoxygénée et isomérisée issue du procédé HDO/ISO est ensuite hydrogénée.

L'hydrogène utilisé dans l'unité d'hydrogénation est typiquement de l'hydrogène hautement purifié. On entend par hautement purifié, de l'hydrogène d'une pureté par exemple supérieure à 99%, même si d'autres grades peuvent également être utilisés.

L'étape d'hydrogénation est effectuée grâce à des catalyseurs. Les catalyseurs d'hydrogénation types peuvent être soit massiques soit supportés et peuvent comprendre les métaux suivants : nickel, platine, palladium, rhénium, rhodium, tungstate de nickel, nickel-molybdène, molybdène, cobalt-molybdène. Les supports peuvent être de la silice, de l'alumine, de la silice-alumine ou des zéolithes.

Un catalyseur préféré est un catalyseur à base de nickel sur support d'alumine dont l'aire de surface spécifique varie entre 100 et 200 m²/g de catalyseur ou un catalyseur massique à base de nickel. Les conditions d'hydrogénation sont typiquement les suivantes :
- Pression : 50 à 160 bars, de préférence 80 à 150 bars et plus préférentiellement 90 à 120 bars ;
- Température : 80 à 180 °C, de préférence 120 à 160 °C et plus préférentiellement 150 à 160 °C ;
- Vitesse volumique horaire (VVH) : 0,2 à 5 hr⁻¹, de préférence 0,4 à 3 hr⁻¹ et plus préférentiellement 0,5 à 0,8 hr¹ ;
- Taux de traitement par l'hydrogène : adapté aux conditions mentionnées ci-dessus et pouvant aller jusqu'à 200 Nm³/tonnes de charge à traiter.

La température dans les réacteurs est typiquement comprise entre 150 et 160°C avec une pression d'environ 100 bars tandis que la vitesse volumique horaire est d'environ 0,6 hr⁻¹ avec un taux de traitement adapté en fonction de la qualité de la charge à traiter et des paramètres du premier réacteur d'hydrogénation.

L'hydrogénation peut avoir lieu dans un ou plusieurs réacteurs en série. Les réacteurs peuvent comprendre un ou plusieurs lits catalytiques. Les lits catalytiques sont généralement des lits catalytiques fixes.

Le procédé d'hydrogénation comprend de préférence deux ou trois réacteurs, de préférence trois réacteurs et est plus préférentiellement réalisé dans trois réacteurs en série.

Le premier réacteur permet le piégeage des composés soufrés et l'hydrogénation d'essentiellement tous les composés insaturés et jusqu'à environ 90 % des composés aromatiques. Le produit issu du premier réacteur ne contient substantiellement aucun composé soufré. Au second stade c'est-à-dire dans le second réacteur, l'hydrogénation des aromatiques se poursuit et jusqu'à 99 % des aromatiques sont de ce fait hydrogénés.

Le troisième stade dans le troisième réacteur est un stade de finition permettant d'obtenir des teneurs en aromatiques inférieures ou égales à 500 ppm, de préférence inférieures ou égales à 300 ppm, préférentiellement inférieures ou égales à 100 ppm, plus préférentiellement inférieures ou égales à 50 ppm, et idéalement inférieures ou égales à 20 ppm même dans le cas de produits à haut point d'ébullition par exemple supérieur à 300°C.

Il est possible d'utiliser un réacteur qui comporte deux ou trois lits catalytiques ou plus. Les catalyseurs peuvent être présents à des quantités variables ou essentiellement égales dans chaque réacteur ; pour trois réacteurs, les quantités en fonction du poids peuvent par exemple être de 0,05-0,5/0,10-0,70/0,25-0,85, de préférence 0,07-0,25/0,15-0,35/0,4-0,78 et plus préférentiellement de 0,10-0,20/0,20-0,32/0,48-0,70.

Il est également possible d'utiliser un ou deux réacteurs d'hydrogénation au lieu de trois.

Il est également possible que le premier réacteur soit composé de réacteurs jumeaux mis en oeuvre de manière alternative. Ce mode d'opérabilité permet notamment un chargement et un déchargement facilité des catalyseurs : lorsque le premier réacteur comprend le catalyseur saturé en premier (substantiellement tout le soufre est piégé sur et/ou dans le catalyseur) il doit être changé souvent.

Un seul réacteur peut également être utilisé dans lequel deux, trois lits catalytiques ou plus sont installés.

Il peut être nécessaire d'insérer des boîtes de quench (au sens anglais « d'étouffement de la réaction ») dans le système de recycle ou entre les réacteurs pour refroidir les effluents d'un réacteur à un autre ou d'un lit catalytique à un autre afin de contrôler les températures et l'équilibre hydrothermique de chaque réaction. Selon un mode de réalisation préféré, il n'y a pas d'intermédiaires de refroidissement ou d'étouffement.

Selon un mode de réalisation, le produit issu du procédé et/ou les gaz séparés sont au moins en partie recyclé(s) dans le système d'alimentation des réacteurs d'hydrogénation. Cette dilution contribue à maintenir l'exothermicité de la réaction dans des limites contrôlées, en particulier au premier stade. Le recyclage permet en outre un échange de chaleur avant la réaction et aussi un meilleur contrôle de la température.

L'effluent de l'unité d'hydrogénation contient principalement le produit hydrogéné et de l'hydrogène. Des séparateurs flash sont utilisés pour séparer les effluents en phase gazeuse, principalement l'hydrogène résiduel, et en phase liquide, principalement les coupes hydrocarbonées hydrogénées. Le procédé peut être effectué en utilisant trois séparateurs flash, un à pression élevée, un à pression intermédiaire et un à basse pression très proche de la pression atmosphérique.

L'hydrogène gazeux qui est recueilli en haut des séparateurs flash peut être recyclé dans le système d'alimentation de l'unité d'hydrogénation ou à différents niveaux dans les unités d'hydrogénation entre les réacteurs.

Selon un mode de réalisation, le produit final est séparé à pression atmosphérique. Il alimente ensuite directement une unité de fractionnement sous vide. De préférence, le fractionnement se fera à une pression comprise entre 10 et 50 mbars et plus préférentiellement à environ 30 mbars.

Le fractionnement peut être effectué de façon à ce qu'il soit possible de retirer simultanément divers fluides hydrocarbonés de la colonne de fractionnement et à ce que leur température d'ébullition puisse être prédéterminée.

En adaptant la charge au travers de ses points d'ébullition initiaux et finaux, les réacteurs d'hydrogénation, les séparateurs et l'unité de fractionnement peuvent donc être directement connectés sans qu'il soit nécessaire d'utiliser des cuves intermédiaires. Cette intégration de l'hydrogénation et du fractionnement permet une intégration thermique optimisée associée à une réduction du nombre d'appareils et à une économie d'énergie.

L'huile hydrocarbonée mise en oeuvre dans la composition épaississante de l'invention est avantageusement une coupe hydrocarbonée ayant un intervalle de distillation ID (en °C) allant de 230°C à 340°C, de préférence de 235°C à 330°C et plus préférentiellement de 240°C à 325°C mesuré selon la norme ASTM D86. De préférence, la différence entre le point d'ébullition initial et le point d'ébullition final est inférieure ou égale à 80°C, préférentiellement inférieure ou égale à 70°C, plus préférentiellement inférieure ou égale à 60°C et avantageusement comprise entre 40 et 50°C. L'huile hydrocarbonée peut comprendre une ou plusieurs fractions d'intervalles de distillation compris dans les intervalles décrits ci-dessus.

Avantageusement, l'huile hydrocarbonée mise en oeuvre dans la composition épaississante de l'invention est totalement saturée. De préférence, les composants de l'huile hydrocarbonée sont choisis parmi les isoparaffines comprenant 12 à 30 atomes de carbone, préférentiellement 13 à 19 atomes de carbone et plus préférentiellement 14 à 18 atomes de carbones.

La composition épaississante selon l'invention comprend avantageusement une teneur en poids en isohexadécane inférieure ou égale à 50%.

L'huile hydrocarbonée de la composition épaississante selon l'invention est idéalement issue du traitement de matières premières d'origine biologique.

L'huile hydrocarbonée de la composition épaississante selon l'invention présente typiquement une teneur en biomatériau d'au moins 90%. Cette teneur est avantageusement plus élevée, en particulier supérieure ou égale à 95%, préférablement supérieure ou égale à 98 % et avantageusement égale à 100%.

En plus d'une teneur particulièrement élevée en biomatériau, l'huile hydrocarbonée de la composition épaississante selon l'invention possède une biodégradabilité particulièrement bonne. La biodégradation d'un produit chimique organique se réfère à la réduction de la complexité des composés chimiques grâce à l'activité métabolique de micro-organismes. Dans des conditions aérobies, les micro-organismes transforment les substances organiques en dioxyde de carbone, eau et biomasse. La méthode OCDE 306, est utilisée pour l'évaluation de la biodégradabilité des substances individuelles dans l'eau de mer. Selon cette méthode, l'huile hydrocarbonée a une biodégradabilité à 28 jours d'au moins 60%, de préférence d'au moins 70%, plus préférablement d'au moins 75% et avantageusement d'au moins 80%.

### Polymère poly(farnesène) :

Le poly(farnesène) mis en oeuvre dans la présente invention est typiquement un polymère du farnesène qui peut être partiellement ou totalement hydrogéné et/ou qui peut être fonctionnalisé, notamment en bout de chaîne, par exemple par des groupements hydroxyle.

Le farnesène existe sous différents isomères, tels que l'alpha-farnesène et le béta-farnesène. Le béta-farnesène peut être représenté par la formule suivante (I):

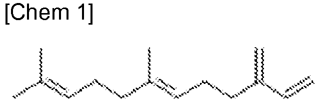

Selon l'invention, le poly(farnesène) présente une masse molaire moyenne en nombre allant de 10000 à 120000 g/mol, de préférence de 20000 à 110000 g/mol, de préférence encore de 30000 à 100000 g/mol, préférentiellement de 40000 à 90000 g/mol, plus préférentiellement de 50000 à 80000 g/mol. Selon un mode de réalisation, le poly(farnesène) présente une masse molaire moyenne en nombre allant de 10000 à 90000 g/mol, de préférence de 20000 à 80000 g/mol. La masse molaire moyenne en nombre peut être mesurée par chromatographie sur gel perméable (en anglais « gel permeation chromatography ») en utilisant des étalons de polystyrène. Le poly(farnesène) utilisé dans l'invention peut présenter une viscosité à 25°C allant de 20000 à 1000000 mPa.s, de préférence de 50000 à 800000 mPa.s, de préférence encore de 100000 à 600000 mPa.s.

Typiquement, le polymère utilisé dans l'invention est un homopolymère du béta-farnesène.

Le poly(farnesène) utilisé selon l'invention peut comprendre n monomères de formule (II) et/ou m monomères de formule (III) :

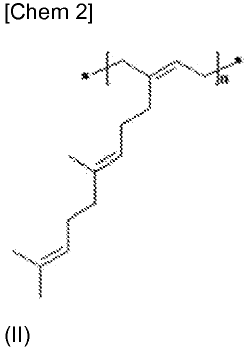

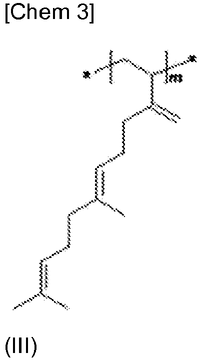

Où n et m sont indépendamment l'un de l'autre des nombres entiers allant par exemple de 40 à 600.

Selon un mode de réalisation particulier, le polyfarnesène utilisé selon l'invention est constitué de monomères de formule (II) et/ou de monomères de formule (III) telles que définies ci-dessus.

Le poly(farnesène) utilisé selon l'invention peut être fonctionnalisé en bout de chaîne, par exemple avec une ou deux fonctions hydroxyle.

Selon un mode de réalisation particulier, le poly(farnesène) utilisé selon l'invention est partiellement ou totalement hydrogéné. Par « poly(farnesène) partiellement hydrogéné », on désigne un poly(farnesène) comprenant au moins une insaturation mais dont une partie des insaturations a été hydrogénée. Par « poly(farnesène) totalement hydrogéné », on désigne un poly(farnesène) saturé, ne comprenant plus d'insaturations.

Selon un mode de réalisation, le polymère utilisé dans l'invention est un poly(farnesène) totalement hydrogéné.

Ainsi, selon un mode de réalisation de l'invention, le polymère poly(farnesène) utilisé selon l'invention comprend n monomères de formule (II bis) et m monomères de formule (III bis) : 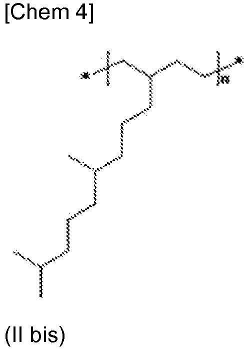 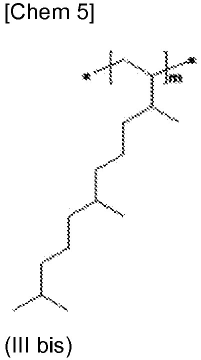 où n et m ont la même signification que ci-dessus.

Selon un mode de réalisation particulier, le poly(farnesène) utilisé selon l'invention est constitué de monomères de formule (II) et/ou de monomères de formule (III) et/ou de monomères de formule (II bis) et/ou de monomères de formule (III bis), telles que définies ci-dessus.

Les inventeurs ont découvert que le poly(farnesène) présentant une masse molaire moyenne en nombre allant de 10000 à 120000 g/mol, en particulier de 10000 à 90000 g/mol, permettait d'améliorer la brillance d'une huile, en particulier d'une huile d'origine biologique. L'amélioration de la brillance peut être évaluée par une augmentation de l'indice de réfraction.

### Procédé de fabrication du polymère poly(farnesène) :

Le poly(farnesène) est obtenu à partir de farnesène d'origine biologique. Le farnesène peut être obtenu à partir d'insectes ou de plantes ou par culture de microorganismes. Le farnesène d'origine biologique est disponible commercialement, par exemple auprès de la société Amyris.

Le poly(farnesène) peut être obtenu par tout procédé connu de polymérisation, par exemple par polymérisation anionique qui permet un contrôle plus précis de la masse moléculaire du polymère obtenu. La polymérisation peut être effectuée en batch ou en continu avec un ajout progressif de l'éventuel initiateur, des monomères et de l'éventuel solvant.

La température lors de la polymérisation peut aller de -80 à 80°C.

Le poly(farnesène) peut être obtenu par un procédé décrit de la page 5, ligne 3 à la page 6, ligne 17 du document WO2018/052709.

### Composition épaississante :

Selon un mode de réalisation de l'invention, la composition épaississante selon la présente invention comprend:
- de 10 à 90% en poids, de préférence de 20 à 80% en poids, préférentiellement de 30 à 70% en poids, plus préférentiellement de 40 à 60% en poids, d'huile(s) d'origine biologique, et
- de 10 à 90% en poids, de préférence de 20 à 80% en poids, préférentiellement de 30 à 70% en poids, plus préférentiellement de 40 à 60% en poids, de polymère(s) poly(farnesène) ayant une masse molaire moyenne en nombre allant de 10000 à 120000 g/mol,
par rapport au poids total de la composition épaississante.

Selon un mode de réalisation de l'invention, la composition épaississante selon la présente invention comprend :
- de 10 à 90% en poids, de préférence de 20 à 80% en poids, préférentiellement de 30 à 70% en poids, plus préférentiellement de 40 à 60% en poids, d'huile(s) hydrocarbonée(s) telle(s) que définie(s) précédemment, et
- de 10 à 90% en poids, de préférence de 20 à 80% en poids, préférentiellement de 30 à 70% en poids, plus préférentiellement de 40 à 60% en poids, de polymère(s) poly(farnesène) ayant une masse molaire moyenne en nombre allant de 10000 à 120000 g/mol,
par rapport au poids total de la composition épaississante.

Selon un mode de réalisation de l'invention, la composition épaississante selon la présente invention comprend:
- de 10 à 90% en poids, de préférence de 20 à 80% en poids, préférentiellement de 30 à 70% en poids, plus préférentiellement de 40 à 60% en poids, d'huile(s) d'origine biologique, et
- de 10 à 90% en poids, de préférence de 20 à 80% en poids, préférentiellement de 30 à 70% en poids, plus préférentiellement de 40 à 60% en poids, de polymère(s) poly(farnesène) ayant une masse molaire moyenne en nombre allant de 40000 à 90000 g/mol,
par rapport au poids total de la composition épaississante.

Selon un mode de réalisation de l'invention, la composition épaississante selon la présente invention comprend:
- de 10 à 90% en poids, de préférence de 20 à 80% en poids, préférentiellement de 30 à 70% en poids, plus préférentiellement de 40 à 60% en poids, d'huile(s) hydrocarbonée(s) telle(s) que définie(s) précédemment, et
- de 10 à 90% en poids, de préférence de 20 à 80% en poids, préférentiellement de 30 à 70% en poids, plus préférentiellement de 40 à 60% en poids, de polymère(s) poly(farnesène) ayant une masse molaire moyenne en nombre allant de 40000 à 90000 g/mol,
   par rapport au poids total de la composition épaississante.

Selon un mode de réalisation de l'invention, la composition épaississante présente une viscosité mesurée à 40°C (mesurée par exemple sur la norme ISO 3104) allant de 300 à 1000 mPa.s.

Ainsi, la composition épaississante selon l'invention comprendra de préférence une seule phase grasse.

La composition épaississante selon l'invention est de préférence non aqueuse. Autrement dit, la composition épaississante selon l'invention comprendra de préférence moins de 10% en poids d'eau, voire moins de 5% en poids d'eau, idéalement moins de 1 % en poids d'eau.

Selon un mode de réalisation de l'invention, la composition épaississante consiste en une ou plusieurs huiles hydrocarbonées telles que définies ci-dessus et en un ou plusieurs polymères poly(farnesène) tels que définis ci-dessus.

### Préparation de la composition épaississante :

La composition épaississante peut être préparée par simple mélange de l'huile ou des huiles d'origine biologique et du polymère poly(farnesène), de préférence à température ambiante.

### Additifs :

La composition épaississante selon l'invention peut également être mise en mélange avec tout adjuvant ou additif habituellement utilisé dans le domaine considéré, notamment cosmétique. Bien entendu, l'homme du métier veillera à choisir le ou les éventuels additifs de la composition selon l'invention de manière telle que les propriétés avantageuses attachées intrinsèquement à la composition épaississante conforme à l'invention ne soient pas ou substantiellement pas, altérées par l'addition envisagée.

Parmi les adjuvants classiques susceptibles d'être contenus (selon le caractère hydrosoluble ou liposoluble de ces adjuvants), on peut citer notamment les tensioactifs moussants anioniques (tels que lauryl ether sulfate de sodium, alkyl phosphate de sodium, tridecyl ether sulfate de sodium), amphotères (tels que alkyl bétaine, disodium cocoamphodiacetate) ou non ioniques de HLB supérieure à 10 (tels que POE/PPG/POE, Alkylpolyglucoside, polyglyceryl-3hydroxylauryl ether) ; les conservateurs tels que le chlorure de benzalkonium; les séquestrants (EDTA) ; les antioxydants ; les parfums ; les matières colorantes telles que les colorants solubles, les pigments et les nacres ; les charges matifiantes, tenseurs, blanchissantes ou exfoliantes ; les filtres solaires ; les actifs cosmétiques ou dermatologiques et agents ayant pour effet d'améliorer les propriétés cosmétiques de la peau, hydrophiles ou lipophiles ; les électrolytes. Les quantités de ces différents adjuvants sont celles classiquement utilisées dans le domaine considéré, et par exemple de 0,01 à 20 % du poids total de la composition. Comme actifs utilisables dans la composition épaississante de l'invention, on peut citer par exemple, les vitamines hydrosolubles ou liposolubles comme la vitamine A (rétinol ou béta-carotène), la vitamine E (tocophérol), la vitamine C (acide ascorbique), la vitamine B5 (panthénol), la vitamine B3 (niacinamide), les dérivés de ces vitamines (notamment esters) et leurs mélanges ; les antiseptiques ; les actifs antibactériens comme le 2,4,4'-trichloro-2'-hydroxy diphényl éther (ou triclosan), le 3,4,4'-trichlorocarbanilide (ou triclocarban) ; les antisébohrréïques ; les antimicrobiens tels que le peroxyde de benzoyle, la niacine (vit. PP) ; les amincissants tels que la caféine ; les azurants optiques, et tout actif approprié pour le but final de la composition, et leurs mélanges.

La composition épaississante peut par exemple comprendre en outre un agent polaire, en particulier dans le cas où l'huile d'origine biologique est une huile hydrocarbonée, i.e. une huile peu polaire. L'agent polaire peut être choisi parmi les triglycérides. Ledit agent polaire peut être ajouté à une teneur allant de 1 à 30% en poids, ou de 5 à 25% en poids, par rapport au poids total de la composition épaississante.

Selon un autre mode de réalisation, la composition épaississante de l'invention ne comprend pas de triglycérides ou comprend des triglycérides en une quantité inférieure à 0,01% en poids, par rapport au poids total de la composition épaississante.

Selon un autre mode de réalisation, la composition épaississante de l'invention ne comprend pas d'agent polaire ou comprend des agents polaires en une quantité inférieure à 0,01% en poids, par rapport au poids total de la composition épaississante.

Selon un mode de réalisation préféré, la composition épaississante de l'invention est essentiellement d'origine biosourcée, autrement dit, elle comprend de préférence au moins 90% en poids, de préférence au moins 95% en poids, de préférence encore au moins 98% en poids, voire au moins 99% en poids, d'ingrédients d'origine biologique, par rapport au poids total de la composition épaississante.

Selon un mode de réalisation de l'invention, la composition épaississante selon l'invention présente un indice de réfraction d'au moins 1,445, de préférence d'au moins 1,450, mesuré par exemple à 25°C selon la norme ASTM D 1218.

La présente invention concerne également l'utilisation de la composition épaississante selon l'invention dans des compositions cosmétiques, en particulier comme épaississant, agent sensoriel, agent filmogène, agent texturant, agent améliorant l'imperméabilité (« waterproof »), agent hydratant, conditionneur, huile visqueuse ou ayant un effet seconde peau ou encore comme agent améliorant la brillance ou comme agent anti-pollution.

### Composition cosmétique :

La présente invention a également pour objet une composition cosmétique comprenant la composition épaississante selon l'invention et :
- au moins un corps gras choisi parmi : les huiles végétales différentes de celles de la composition épaississante le cas échéant, les beurres végétaux, les éthers et alcools gras, les esters huileux, les alcanes et les huiles silicones, de préférence parmi les esters huileux,
   et/ou
- au moins un additif choisi parmi les additifs précités, de préférence parmi les tensioactifs moussants anioniques (tels que lauryl éther sulfate de sodium, alkyl phosphate de sodium, tridécyl ether sulfate de sodium), amphotères (tels que alkyl bétaine, disodium cocoamphodiacétate) ou non ioniques de HLB supérieure à 10 (tels que POE/PPG/POE, Alkylpolyglucoside, polyglyceryl-3hydroxylauryl éther).

Le corps gras est de préférence choisi parmi les corps gras présentant une faible polarité.

Des exemples d'huiles végétales sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ou de camélia.

Les beurres végétaux sont des corps gras qui ont les mêmes propriétés que les huiles végétales. La différence entre les deux consiste dans le fait que les beurres se présentent sous forme solide à température ambiante. Aussi, contrairement aux huiles végétales, la matière première dont est extrait un beurre (pulpe, graines ou amandes) est chauffée après avoir été broyée pour l'extraction de la matière grasse. Comme les huiles végétales, les beurres peuvent être raffinés pour assurer une meilleure conservation, neutraliser les odeurs, améliorer la couleur et la consistance. Riches en antioxydants et nourrissants, les propriétés cosmétiques des beurres végétaux améliorent l'élasticité de la peau, protègent des agressions extérieures en laissant un film protecteur sur l'épiderme et réduisant ainsi la déshydratation, réparent et apaisent en régénérant le film hydrolipidique naturel de la peau. Des exemples de beurres végétaux sont notamment le beurre de karité, le beurre de cacao, le beurre de mangue, le beurre de shorea ou encore le beurre d'olive.

Les éthers et les alcools gras sont des substances cireuses grasses à longue chaîne et aux propriétés remarquables notamment filmogènes, émollientes, hydratantes, adoucissantes et protectrices. Ils agissent comme huiles hydratantes et comme émulsifiants. Des exemples d'alcool gras ou d'éthers sont : le cetyl Alcohol, le Stearyl Alcohol, le myristyl alcohol, le lauryl alcohol, le behenyl alcohol, le cetearyl alcohol, les dicaprylyl éthers, les stearyl éthers ou l'octyldodecanol (identifiés par leur dénomination INCI).

Les esters huileux ou huiles estérifiées sont le produit d'une réaction entre des acides gras (acides à chaînes plus longues, comme par exemple l'acide stéarique, l'acide oléique, l'acide palmitique) et des alcools (des alcools gras ou des polyols comme le glycérol). Ces huiles peuvent contenir des substances issues de la pétrochimie, comme c'est le cas pour l'Isopropyl Palmitate. Des exemples d'esters huileux sont le caprylic capric triglycéride, le coco caprylate caprate, l'oleyl erucate, l'oleyl linoleate, le decyl oleate ou encore le PPG-3 benzyl éther myristate (identifiés par leur dénomination INCI).

On entend par huiles de silicones ou polysiloxanes, une huile comprenant au moins un atome de silicium, et notamment au moins un groupe Si-O. Comme huile de silicone, on peut notamment citer le phenylpropyldimethylsiloxysilicate, les dimethicones ou encore le cyclopentasiloxane (identifiés par leur dénomination INCI).

Cette composition cosmétique comprend un milieu physiologiquement acceptable, c'est-à-dire qui ne présente pas d'effets secondaires délétères et en particulier qui ne produit pas de rougeurs, d'échauffements, de tiraillements ou de picotements inacceptables pour un utilisateur. Ce milieu comprend éventuellement de l'eau et/ou au moins une huile en tant que corps gras, en plus de la composition épaississante précitée.

Selon un mode de réalisation, la composition cosmétique a une teneur en composition épaississante telle que décrite ci-dessus allant de 1 à 80%, de préférence de 5 à 70% et avantageusement de 10 à 50% en poids par rapport au poids total de la composition.

Selon un mode de réalisation, la composition cosmétique a une teneur en composition épaississante telle que décrite ci-dessus allant de 1 à 50% en poids, de préférence de 2 à 40% en poids et avantageusement de 3 à 30% en poids, par rapport au poids total de la composition.

Ainsi, la composition cosmétique selon l'invention peut comprendre de 0,1 à 15% en poids de poly(farnesène), de préférence de 0,5 à 10% en poids de poly(farnesène) et avantageusement de 1 à 5% en poids de poly(farnesène), par rapport au poids total de la composition cosmétique, ledit poly(farnesène) ayant de préférence une masse molaire moyenne en nombre allant de 20000 à 90000 g/mol.

La composition cosmétique selon l'invention peut ainsi être une composition anhydre, une émulsion telle qu'une émulsion eau-dans-huile (E/H), une émulsion huile-dans-eau (H/E) ou une émulsion multiple (notamment E/H/E ou H/E/H), une nano-émulsion, ou encore une dispersion, selon les additifs éventuellement introduits et/ou selon une phase aqueuse éventuellement introduite.

La composition cosmétique selon l'invention peut constituer par exemple une composition de soin des lèvres, tel que le gloss ou les sticks hydratants pour les lèvres, une composition de masque, une composition de baume réparateur, une composition gommante et/ou exfoliante aussi bien pour le visage que pour les mains (lorsqu'elle contient des particules exfoliantes), une composition de maquillage, une composition de rasage, une composition de baume après-rasage, une composition parfumée, une composition pour lingettes, une composition de démaquillage ou de nettoyage de la peau, des lèvres, une composition solaire (protection contre les U.V.), ou après-solaire, une composition pour le massage de la peau, une composition de baume soin douche, une composition anti-transpirante.

La composition selon l'invention peut être une composition solaire pour une application topique, comprenant (i) au moins un filtre solaire, tel qu'un anti UVA et/ou un anti UVB, et (ii) au moins une composition épaississante selon l'invention, de préférence en une quantité allant de 1 à 50%, préférentiellement de 2 à 40% et avantageusement de 3 à 30% en poids par rapport au poids total de la composition solaire.

La composition de l'invention se caractérise avantageusement par le fait qu'elle présente une stabilité d'une durée supérieure ou égale à 4 semaines, avantageusement supérieure ou égale à 6 semaines, la stabilité étant évaluée après un stockage sans agitation à température ambiante, à 40°C et à 50°C et correspondant à une évaluation visuelle de la coloration et de l'aspect ainsi qu'une évaluation olfactive et/ou une mesure de la viscosité.

### Utilisation de la composition épaississante :

L'invention a encore pour objet l'utilisation cosmétique de la composition telle que définie ci-dessus pour une application topique, sur la peau, les lèvres ou les phanères (incluant les ongles, le cuir chevelu et les cheveux).

L'invention a aussi pour objet l'utilisation cosmétique de la composition cosmétique telle que définie ci-dessus comme produit de soin de la peau (sérums, crèmes, baumes, etc), comme produit d'hygiène, comme produit solaire/après-solaire, comme produit de maquillage, comme produit démaquillant, comme produit parfumé, comme produit anti-transpirant, comme produit de soin des lèvres, tel que le gloss ou les sticks hydratants pour les lèvres.

La composition épaississante selon l'invention permet d'obtenir une meilleure perception de la couleur de la composition cosmétique la comprenant, ce qui permet de réduire la quantité de pigment utilisée dans ladite composition cosmétique.

La composition cosmétique selon l'invention présente des propriétés d'hydratation améliorées, ainsi que des propriétés anti-pollution and une brillance améliorée.

La composition épaississante selon l'invention et la composition cosmétique selon l'invention peuvent tout particulièrement être utilisées pour une application topique, notamment comme produit de soin pour la peau ou des cheveux, comme produit de maquillage, comme produit capillaire, comme produit démaquillant, comme produit parfumé, comme produit solaire, comme produit de soin des lèvres, tel que le gloss ou les sticks hydratants pour les lèvres.

L'invention a encore pour objet un procédé cosmétique de traitement de la peau, des lèvres ou des phanères, comprenant au moins une étape d'application sur la peau, les lèvres et/ou les phanères d'une composition telle que définie ci-dessus.

La composition de l'invention peut également être utilisée pour la formulation de compositions cosmétiques comprenant d'autres composants ou d'autres phases que ceux décrits ci-dessus. Il peut s'agir notamment de la formulation de compositions de soin, d'hygiène, de maquillage.

### Procédé de traitement cosmétique :

Enfin, l'invention couvre aussi un procédé de traitement cosmétique comprenant au moins une étape d'application, de préférence par étalement, sur la peau, les lèvres ou les phanères, des compositions selon l'invention.

### EXEMPLES

Dans la suite de la présente description, des exemples sont donnés à titre illustratif de la présente invention et ne visent en aucun cas à en limiter la portée.

### Exemple 1 : Préparation des compositions épaississantes :

Le poly(farnesène) utilisé dans les exemples 1 à 5 est totalement hydrogéné et fonctionnalisé avec des hydroxyles. Il présente une masse molaire moyenne en nombre de 71000 g/mol. Il a été préparé selon le mode opératoire décrit dans le document WO2018/052709.

Le tableau 1 regroupe les propriétés physico chimiques de l'huile hydrocarbonée utilisée dans les exemples.

### [Table 1]

**Tableau 1 : propriétés physico chimiques de l'huile hydrocarbonée**

| Caractéristiques | Huile hydrocarbonée |
|---|---|
| Aromatiques (ppm) | <20 |
| Soufre (ppm) | 0,11 |
| % iso paraffines (w/w) | 96,2 |
| % n-paraffines (w/w) | 3,8 |
| % naphténiques (w/w) | 0 |
| C13 (iso) | 0 |
| C14 (iso) | 0 |
| C15 (iso) | 0 |
| C16 (iso) | 1,58 |
| C17 (iso) | 14,17 |
| C18 (iso) | 79,69 |
| C19 (iso) | 0,12 |
| C20 (iso) | 0,38 |
| C27 (iso) | 0,29 |
| Quantité de carbones d'origine biologique (%) | > 98 |
| Point d'ébullition initial (°C) | 293,6 |
| Point ébullition 5% (°C) | 296,7 |
| Point ébullition 50% (°C) | 298,5 |
| Point ébullition 95% (°C) | 305,3 |
| Point d'ébullition final (°C) | 324,1 |
| Biodégradabilité OCDE (28 jours) (%) | 83 |
| Indice de réfraction à 25°C | 1,4394 |
| densité à 15°C (kg/m3) | 787,2 |
| Point éclair (°C) | 149 |
| Viscosité Cinématique à 40°C (cSt) | 3,87 |
| Pression de vapeur à 20°C (kPa) | <0,01 |
| Point d'Aniline (°C) | 93,2 |

Les normes et méthodes suivantes ont été utilisées pour mesurer les propriétés ci-dessus :
- point éclair : EN ISO 2719
- densité à 15°C : EN ISO 1185
- viscosité à 40°C : EN ISO 3104
- point d'aniline : EN ISO 2977
- Point d'ébullition : ASTM D86
- biodégradabilité : méthode OCDE 306
- indice de réfraction à 20°C : ASTM D 1218
- pression de vapeur : calculée selon des méthodes bien connues de l'homme du métier.

Les compositions épaississantes testées sont exposées dans le tableau 2 ci-dessous. Les pourcentages sont des pourcentages en poids par rapport au poids total de la composition épaississante.

### [Table 2]

**Tableau 2 : Compositions épaississantes**

| | Composition 1 | Composition 2 | Composition 3 | Composition 4 |
|---|---|---|---|---|
| Huile hydrocarbonée | 40% | 20% | 60% | 30% |
| Poly(farnesène) | 60% | 60% | 40% | 30% |
| Triglycérides | 0 | 20% | 0 | 40% |

### Exemple 2 : Evaluation de la miscibilité de la composition épaississante avec des corps gras

Les corps gras testés sont :
- une huile végétale : meadowfoam seed oil (selon la dénomination INCI) présentant une densité à 20°C de 0,91 et comprenant :
   ∘ 58,0-64,0% en poids d'acide gadoléique (C20 : 1)
   ∘ 10,0-14,0% en poids d'acide érucique (C22:1 d13)
   ∘ 3,0-6,0% en poids d'acide docosénoïque (C22:1 d5)
   ∘ 15,0-21,0% en poids d'acide docosadiénoïque (C22:2)

Par rapport au poids total de l'huile végétale.
- Une cire végétale : huile de jojoba.
- Un ester huileux : isononyl isononanoate (selon la dénomination INCI), disponible commercialement.
- Une huile silicone : cyclopentasiloxane (selon la dénomination INCI), disponible commercialement.
- Du squalane (selon la dénomination INCI).

La manipulation consiste à observer la miscibilité de la composition épaississante (Compositions 1 et 2 préparées à l'exemple 1) avec les corps gras, en procédant à l'ajout du corps gras à tester dans la composition épaississante par pourcentage en poids croissant : 10%, 25%, 50%, 75% et 90%. Ces manipulations sont réalisées en continu. On observe le mélange entre chaque ajout. L'ajout est effectué dans le même récipient.

Les expériences sont réalisées sur une base de départ de 45g de composition épaississante pour finir à 450g. En effet, les ajouts sont effectués comme tel sur la base de 45g présente dans le bécher :
- 10% : ajout de 5g de corps gras à tester.
- 25% : ajout de 10g de corps gras à tester dans le mélange précédemment obtenu ((5+10)/(45+5+10) = 0,25).
- 50% : ajout de 30g de corps gras à tester dans le mélange précédemment obtenu.
- 75% : ajout de 90g de corps gras à tester dans le mélange précédemment obtenu.
- 90% : ajout de 270g de corps gras à tester dans le mélange précédemment obtenu.

Les mélanges finaux sont conservés dans des pots en verre et observés à J+1. Un mélange est dit non miscible lorsque des sédiments sont visuellement observés.

La composition épaississante selon l'invention est miscible avec l'huile silicone, lorsque l'huile silicone est présente en une quantité allant jusqu'à 50% en poids.

La composition épaississante selon l'invention est miscible avec les autres corps gras testés, lorsque le corps gras est présent en une quantité allant jusqu'à 90% en poids.

### Exemple 3 : Analyse sensorielle de compositions cosmétiques comprenant la composition épaississante selon l'invention.

Quatre compositions cosmétiques ont été préparées et testées. Les compositions cosmétiques sont décrites dans le tableau 3 ci-dessous. Les pourcentages sont des pourcentages en poids par rapport au poids total de la composition cosmétique.

### [Table 3]

**Tableau 3 : Compositions cosmétiques**

| | Placébo | Composition A | Composition B | Composition C | Composition D |
|---|---|---|---|---|---|
| Composition épaississante 3 (de l'exemple 1) | 0 | 15% | 0 | 30% | 0 |
| Composition épaississante 4 (de l'exemple 1) | 0 | 0 | 15% | 0 | 30% |
| DUB MCT 55/45 | 35% | 35% | 35% | 35% | 35% |
| DUB IPP | 35% | 35% | 35% | 35% | 35% |
| DUB ININ | 30% | 15% | 15% | 0 | 0 |

Les compositions sont évaluées, du point de vue sensoriel et organoleptique, par un panel sensoriel formé d'experts (5 personnes).

L'analyse sensorielle et organoleptique s'est déroulée comme suit :
- Dépôt d'une goutte du produit à évaluer sur la peau (dessous de l'avant-bras), à l'aide d'une pipette en verre.
- Application du produit à l'aide des doigts : évaluation de l'aspect sur la peau (brillance), du ressenti (sensoriel) à l'application, et du toucher final après application.

Une note allant de 0 à 3,5 est donnée pour les deux critères : la brillance sur la peau et le gras. Plus la note est élevée, meilleure est la sensation. Les notes sont indiquées dans le tableau 4 ci-dessous.

### [Table 4]

**Tableau 4 : Analyse sensorielle des compositions cosmétiques**

| | Placébo | Composition A | Composition B | Composition C | Composition D |
|---|---|---|---|---|---|
| Brillance sur la peau | 1,5 | 1,7 | 2,7 | 2 | 2,4 |
| Gras | 2,1 | 2,2 | 2,9 | 2,2 | 2,4 |

Comme illustré dans le tableau 4, les compositions cosmétiques comprenant une composition épaississante selon l'invention présentent des notes très satisfaisantes en termes de brillance sur la peau et de gras.

### Exemple 4 : Analyse sensorielle de compositions cosmétiques sous forme d'émulsions comprenant la composition épaississante selon l'invention

Trois compositions cosmétiques sous forme d'émulsion ont été préparées et testées. La composition des émulsions est décrite dans le tableau 5 ci-dessous où les pourcentages sont exprimés en pourcentage en poids par rapport au poids total de l'émulsion.

### [Table 5]

**Tableau 5 : Composition des émulsions à 5% et 10% de composition épaississante selon l'invention**

| Phase de préparation | Nom commercial | Ingrédient | Teneur en % en poids | | |
|---|---|---|---|---|---|
| | | | Placebo | Emulsion 10% | Emulsion 5% |
| A | SIMULSOLO 165 | PEG-100 Stéarate & Glyceryl Stéarate | 3,50 | 3,50 | 3,50 |
| | MONTANOLO 68 | CETEARYL ALCOHOL CETEARYL GLUCOSIDE 0,50GLUCOSE AQUA | 1,50 | 1,50 | 1,50 |
| | TEGOALKANOLO 1618 | CETEARYL ALCOHOL | 0,50 | 0,50 | 0,50 |
| | KARITEO CP | BUTYROSPERMUM PARKII BUTTER | 2,00 | 2,00 | 2,00 |
| | DUB DSPE | Pentaerythrityl Distearate | 0,60 | 0,60 | 0,60 |
| | Composition épaississante 3 ou 4 | | 0 | 10,00 | 10,00 |
| | DUBO ININ | Isononyl Isononanoate | 10,00 | 10,00 | 5,00 |
| B | EAU DEMINERALISEE | AQUA | 74,15 | 64,15 | 69,15 |
| | CARBOPOL ULTREZO 21 | Acrylates/C10-30 Alkyl Acrylate Crosspolymer | 0,20 | 0,20 | 0,20 |
| | GLYCERINE CODEX | GLYCERIN | 3,00 | 3,00 | 3,00 |
| | RHODICAREO T | XANTHAN GUM AQUA | 0,20 | 0,20 | 0,20 |
| C | PHENOXETOL | PHENOXYETHANOL | 0,80 | 0,80 | 0,80 |
| | SOLUTION SOUDE A 10% | SOUDE | 0,30 | 0,30 | 0,30 |
| D | EAU DEMINERALISEE | AQUA | 2,00 | 2,00 | 2,00 |
| | HYDROLITE^{®}-5 | Pentylene Glycol | 1,00 | 1,00 | 1,00 |
| | CHLORPHENESINO BP73 | CHLORPHENESIN AQUA | 0,25 | 0,25 | 0,25 |

Chaque émulsion est préparée de la manière suivante :
- Préparation de la phase A en pesant les ingrédients et en mettant sous agitation et chauffage à 75°C les ingrédients jusqu'à l'obtention d'une phase homogène.
- Préparation de la phase B :
   ∘ Peser l'eau de la phase B.
   ∘ Sans agitation, saupoudrer le carbopol Ultrez 21 et laisser hydrater 10 minutes sans agitation.
   ∘ Mettre sous agitation et chauffer à 75°C. Durant la chauffe, sous vive agitation verser le prémix de la Rhodicare-t dans la Glycérine.
   ∘ Maintenir sous agitation jusqu'à l'obtention d'une phase homogène, tout en poursuivant la chauffe à 75°C.
- A 75°C, sous vive agitation, réaliser l'émulsion en versant la phase A dans la phase B.
- Commencer le refroidissement en maintenant la vitesse d'agitation. A 72°C homogénéiser pendant 30 secondes avec un disperseur turrax^{®} réglé à 9500rpm et poursuivre le refroidissement sous agitation modérée.
- A 50°C, sous agitation, introduire successivement les ingrédients de la phase C et poursuivre le refroidissement sous agitation modérée.
- A 30°C, sous agitation, introduire successivement les composants de la phase D.
- Mesurer le pH et l'ajuster si nécessaire : 5,00 < pH < 6,00.

Les compositions sont évaluées, du point de vue sensoriel et organoleptique, par un panel sensoriel formé d'experts (5 personnes).

L'analyse sensorielle et organoleptique s'est déroulée comme suit :
- Dépôt d'une goutte du produit à évaluer sur la peau (dessous de l'avant-bras), à l'aide d'une pipette en verre, et analyse de l'étalement de la goutte.
- Application du produit à l'aide des doigts : évaluation de l'étalement et de l'aspect sur la peau (brillance).

Une note allant de 0 à 3,5 est donnée pour les deux critères : la brillance sur la peau et l'étalement. Plus la note est élevée, meilleure est la sensation. Les notes sont indiquées dans le tableau 6 ci-dessous.

### [Table 6]

**Tableau 6 : Analyse sensorielle des émulsions selon l'invention**

| | Placébo | Emulsion 10% avec composition épaississante 3 | Emulsion 5% avec composition épaississante 3 | Emulsion 5% avec composition épaississante 4 |
|---|---|---|---|---|
| Brillance sur la peau | 0,4 | 0,9 | 0,5 | 0,8 |
| Etalement | 2,7 | 3,5 | 3,1 | 3,2 |

Comme illustré dans le tableau 6, les émulsions comprenant une composition épaississante selon l'invention présentent des notes très satisfaisantes en termes de brillance sur la peau et d'étalement.

### Exemple 5 : Détermination de l'indice de réfraction

L'indice de réfraction des compositions épaississantes 1 à 4 selon l'invention (décrites dans le tableau 2 ci-dessus) a été déterminé et comparé à l'indice de réfraction de l'huile hydrocarbonée (décrite dans le tableau 1 ci-dessus).

L'indice de réfraction a été mesuré à 25°C selon la norme ASTM D 1218 et est indiqué dans le tableau 7 ci-dessous.

### [Table 7]

**Tableau 7 : Indice de réfraction**

| | Composition 1 | Composition 2 | Composition 3 | Composition 4 | Huile hydrocarbonée |
|---|---|---|---|---|---|
| Indice de réfraction | 1,461 | 1,466 | 1,454 | 1,462 | 1,439 |

L'indice de réfraction d'une composition est un indicateur de la brillance de ladite composition. Ainsi, plus l'indice de réfraction d'une composition est élevé, plus la composition est brillante. Comme le montre le tableau 7, les compositions épaississantes selon l'invention présentent un indice de réfraction élevée, ce qui prouve que les compositions épaississantes selon l'invention sont très brillantes.

### Exemple 6 : Préparation d'autres compositions épaississantes :

Le poly(farnesène) utilisé dans la suite des exemples est totalement hydrogéné et non fonctionnalisé par des hydroxyles. Il présente une masse molaire moyenne en nombre de 71000 g/mol. Il a été préparé selon le mode opératoire décrit dans le document WO2018/052709, à l'exception de l'introduction d'un groupe fonctionnel.

L'huile d'origine biologique utilisée dans la suite des exemples et l'huile hydrocarbonée décrite dans l'exemple 1.

Les compositions épaississantes testées sont exposées dans le tableau 8 ci-dessous. Les pourcentages sont des pourcentages en poids par rapport au poids total de la composition épaississante.

**[Table 8]**

| | Composition 5 | Composition 6 |
|---|---|---|
| Huile hydrocarbonée | 40% | 60% |
| Poly(farnesène) | 60% | 40% |

### Exemple 7 : Produits de maquillage ou produit solaire

Dans cet exemple, les propriétés de résistance à l'eau (water-proof) et les propriétés dispersant de pigment de couleur de compositions à des fins d'applications maquillage ou produit solaire ont été évaluées.

Les compositions évaluées sont décrites dans le tableau 9 ci-dessous. Les pourcentages sont des pourcentages en poids par rapport au poids total de la composition.

**[Table 9]**

| | Nom commercial | ingrédient | Teneur en % en poids | | | | | |
|---|---|---|---|---|---|---|---|---|
| | | | Ex 7.1 | Ex 7.2 | Ex 7.3 | Ex 7.4 | Ex 7.5 | Ex 7.6 |
| A | | Composition épaississante 5 (exemple 6) | 42,5 | | | 10 | | |
| | | Composition épaississante 6 (exemple 6) | | 42,5 | | | | |
| | PANALANE H-300E | Polyisobutène hydrogéné | | | 42,5 | | 10 | |
| | XIAMETER PMX 1502-FLUID | C11-13 isoparaffine et diméthiconol & Isohexadécane & Diméthicone | | | | | | 10 |
| | EMOGREEN L19 | C15-19 alkane | 8 | 8 | 8 | 28 | 28 | 28 |
| | BENTONE GEL VS-5PC V | Cyclopentasiloxane & disteardimonium Hectorite & Propylene carbonate | 30 | 30 | 30 | | | |
| | BENTONE GEL IHD V | Isohexadécane & disteardimonium Hectorite & Propylene carbonate | | | | 30 | 30 | 30 |
| B | CREASPERSE GERANIUM (CPO63) | Polydécène hydrogéné & Red 7 Lake (Cl 15850) & acide poly-hydroxystéarique | 15 | 15 | 15 | | | |
| | CREASPERSE WHITE R (CPO78) | Dioxide de titane & Polydécène hydrogéné et acide hydroxystéarique | 3 | 3 | 3 | | | |
| | KAHLWAX 8104 BEESWAX WHITE | Cera Alba | | | | 12 | 12 | 12 |
| | CARNAUBA WAX SP-63 | Copernicia Cerifera (Carnauba) Wax | | | | 6 | 6 | 6 |
| | CUTINA GMS V | Glyceryl Stéarate | | | | 3 | 3 | 3 |
| C | FRUITS ROUGES FML00100 | Parfum | 1,5 | 1,5 | 1,5 | | | |
| | UNIPURE BLACK LC 989 HLC | CI 77499 & Lécithine hydrogénée | | | | 10 | 10 | 10 |
| | LEXGARD GMCY | Glycéryl Caprylate | | | | 1 | 1 | 1 |

Les compositions Ex7.1, Ex7.2 et Ex7.3 sont évaluées en termes de propriétés dispersant de pigment de couleur par analyse colorimétrique, selon le système d'acceptabilité CMC.

Comme illustré dans le tableau 10 ci-dessous, les compositions Ex7.1 et Ex7.2 selon l'invention présentent une couleur plus stable (voir la valeur de dE-CMC) que la composition comparative Ex7.3.

**[Table 10]**

| | L* | a* | b* | C* | h | dE-CMC(l :c) |
|---|---|---|---|---|---|---|
| Ex7.1 | 34,1 | 50,26 | 15,57 | 52,61 | 17,21 | 0,65 |
| Ex7.2 | 34,02 | 49,75 | 15,38 | 52,07 | 17,18 | 0,52 |
| Ex7.3 | 33,8 | 49,69 | 14,63 | 51,8 | 16,41 | - |

La composition épaississante selon l'invention permet d'avoir de bonnes propriétés colorimétriques, en particulier de maintien de la couleur, ce qui lui permet d'être utilisée de manière satisfaisante dans des compositions de maquillage par exemple.

On note également une couleur plus saturée/plus intense pour les Ex7.1 et Ex7.2 (critère C). Ceci améliore la perception de la couleur ce qui pourrait permettre de réduire le taux de pigment utilisé dans la composition cosmétique.

Les compositions Ex7.4, Ex7.5, Ex7.6 sont évaluées en termes de résistance à l'eau par un essai clinique sur 11 personnes volontaires. Chaque composition est appliquée sur les cils de chaque volontaire, puis laissée séchée pendant 5 minutes avant exposition du visage à de la vapeur (d'un Vapozone) pendant 10 minutes.

Des notes de 0 à 4 sont accordées par des professionnels en fonction des traces de bavures de la composition après 10 minutes d'exposition à la vapeur. Les notes sont attribuées de la façon suivante :
- 4 si aucune bavure,
- 3 si très légère bavure,
- 2 si légère bavure,
- 1 si bavure moyenne,
- 0 si importante bavure.

Un pourcentage de rémanence est calculé à partir de la moyenne des notes attribuées de la façon suivante : %rémanence = moyenne des notes/4 * 100. Ce pourcentage quantifie la résistance à l'eau.

Il a été observé que la composition selon l'invention Ex7.4 présente une bien meilleure résistance à l'eau que les compositions comparatives Ex7.5 et Ex7.6. En effet, la composition Ex7.4 présente plus de 75% de rémanence alors que les compositions Ex7.5 et Ex7.6 présentent respectivement seulement environ 45% et environ 52% de rémanence.

La composition épaississante selon l'invention permet d'améliorer les propriétés de résistance à l'eau de compositions, telles que des compositions cosmétiques ou des compositions de protection solaire.

### Exemple 8 : Produits capillaires

Dans cet exemple, les propriétés sensorielles de compositions à des fins d'applications capillaires ont été évaluées.

Les compositions évaluées sont décrites dans le tableau 11 ci-dessous. Les pourcentages sont des pourcentages en poids par rapport au poids total de la composition.

**[Table 11]**

| | Nom commercial | ingrédient | Teneur en % en poids | | |
|---|---|---|---|---|---|
| | | | Ex8.1 | Ex8.2 | Ex8.3 |
| A | VARISOFTO BTMS | | 8 | 8 | 8 |
| | CUTINAO PES | | 2 | 2 | 2 |
| | KARITEO CP | BUTYROSPERMUM PARKII BUTTER | 10 | 10 | 10 |
| | EMOSMART^{®} L19 | | 5 | 5 | 5 |
| | | Composition épaississante 5 (exemple 6) | 5 | | |
| | | Composition épaississante 6 (exemple 6) | | 5 | |
| | XIAMETER PMX 1503 FLUID | | | | 5 |
| B | EAU DEMINERALISEE | AQUA | 62,82 | 62,82 | 62,82 |
| | GLYCERINE CODEX | GLYCERIN | 3 | 3 | 3 |
| C | CHLORPHENESINO BP73 | CHLORPHENESIN AQUA | 0,28 | 0,28 | 0,28 |
| D | PHENOXETOL | PHENOXYETHANOL | 0,9 | 0,9 | 0,9 |
| | ISOPENTYLDIOL | | 3 | 3 | 3 |

Les compositions Ex8.1, Ex8.2, Ex.8.3 sont évaluées en termes de souplesse, de brillance, de sensation de cheveux nourris par un essai clinique sur 3 personnes volontaires. Chaque composition est appliquée sur les cheveux de chaque volontaire, puis laissée séchée.

Des notes allant de 1 à 5 sont données pour chaque critère : souplesse des cheveux après séchage et sensation de cheveux nourris. Plus la note est élevée meilleure est la propriété.

Les notes sont indiquées dans le tableau 12 ci-dessous.

**[Table 12]**

| | Ex8.1 | Ex8.2 | Ex8.3 |
|---|---|---|---|
| Souplesse des cheveux après séchage | 2,8 | 2,8 | 2,3 |
| Sensation de cheveux nourris | 3,0 | 3,6 | 2,6 |

La composition épaississante selon l'invention permet d'améliorer les propriétés nourrissantes d'une composition capillaire, telle qu'un masque capillaire.

### Exemple 9 : Compositions de soin de la peau

Dans cet exemple, les propriétés filmogène (« film-forming » en anglais) induisant un effet hydratant et les propriétés anti-pollution des compositions épaississantes selon l'invention ont été évaluées.

Les compositions évaluées sont décrites dans le tableau 13 ci-dessous. Les pourcentages sont des pourcentages en poids par rapport au poids total de la composition.

**[Table 13]**

| Phase de préparation | INCI ou composition invention | Teneur en % en poids | | |
|---|---|---|---|---|
| | | Ex9.1 | Ex9.2 | Ex9.3 |
| A | Steareth-2 | 3 | 3 | |
| | Cetyl Alcohol & Glyceryl Stéarate & Peg-75 Stéarate & Ceteth-20 & Steareth 20 | | | 5 |
| | Steareth-21 | 2 | 2 | |
| | PPG-15 Stearyl Ether | 2 | 2 | |
| | Cetearyl Alcohol | 1,5 | 1,5 | |
| | BUTYROSPERMUM PARKII BUTTER | 1 | 1 | |
| | C10-18 Triglycérides | 2 | 2 | 4 |
| | Glycol Palmitate & Glycol & Palmitic Acid | | | 4,5 |
| | Theobroma Cacao Seed Butter | | | 2 |
| | Macadamia Integrifolia Seed Oil | 1,5 | 1,5 | |
| | C15-19 Alkane | 2 | 2 | 7 |
| | Composition épaississante 5 (exemple 6) | 5 | | 5 |
| | Dimethicone (Xiameter PMX-200 SIL FLUID 1000CTS) | | 5 | |
| B | AQUA | 72,8525 | 72,8525 | 65,25 |
| | Disodium EDTA | | | 0,1 |
| | GLYCERIN | 3 | 3 | 4 |
| | Acrylates/C10-30 Alkyl Acrylate Crosspolymer | 0,25 | 0,25 | 0,2 |
| | Xanthan gum | | | 0,15 |
| | Pentylene glycol | | | 1 |
| | Chlorphenesin | | | 0,2 |
| C | PHENOXYETHANOL | 0,8 | 0,8 | |
| | AQUA et Sodium Hydroxide | 0,5475 | 0,5475 | 0,45 |
| D | AQUA | 1 | 1 | |
| | Phenoxyethanol | | | 0,8 |
| | Pentylene Glycol | 1 | 1 | |
| | CHLORPHENESIN | 0,25 | 0,25 | |
| E | Parfum | 0,3 | 0,3 | 0,35 |

Les propriétés filmogènes ont été évaluées en évaluant la perte insensible en eau (PIE) sur une durée de 2 heures (en anglais « Transepidermal water loss » ou TEWL), pendant un essai clinique sur 11 volontaires. La Perte Insensible en Eau (PIE) est la mesure la plus couramment utilisée pour quantifier les échanges peau/milieu extérieur. Cette mesure permet d'évaluer la quantité d'eau évaporée par la peau, elle est exprimée en grammes par heure et par m² de surface cutanée. La mesure est effectuée par une sonde Tewamètre TM300 (Courage & Khazaka GmbH).

Cette étude a consisté à mesurer le taux de PIE avant l'application (T0) et 2 heures (T2h) après application de la composition au niveau des mains, sur une zone à traiter et sur une zone témoin. La composition est appliquée sur une zone de 16 cm² à raison de 2 mg/cm² au niveau de la zone à traiter et une zone témoin (non revêtue de la composition) de 16 cm² est également délimitée. Le tableau 14 ci-dessous rassemble les taux de PIE (moyenne sur les 11 volontaires) en g/m²/h à T0 et à T2h entre la zone traitée et la zone témoin pour chacune des deux compositions testées (Ex9.1 et Ex9.2). Le pourcentage de variation entre T0 et T2h est également indiqué dans le tableau 14.

**[Tableau 14]**

| | T0 | | T2h | |
|---|---|---|---|---|
| | Zone traitée | Zone témoin | Zone traitée | Zone témoin |
| Moyenne PIE (g/m²/h) - Ex9.1 | 12,9 | 12,3 | 10,7 | 12,7 |
| Variation - Ex9.1 | 5,2% | | -15,9% | |
| Moyenne PIE (g/m²/h) - Ex9.2 | 8,7 | 8,8 | 5,8 | 6,7 |
| Variation (%) - Ex9.2 | -1,1% | | -14,5% | |

Les résultats montrent une diminution de la perte d'eau de presque 16% grâce à la composition Ex9.1 selon l'invention. En comparaison, la composition hors invention Ex9.2 (diméthicone) donne une diminution de la perte d'eau de 14,5%.

La diminution significative de la perte d'eau grâce à la composition épaississante selon l'invention montre un effet filmogène et donc une amélioration des propriétés hydratantes grâce à la composition épaississante selon l'invention.

Les propriétés anti-pollution de la composition Ex9.3 comprenant la composition épaississante selon l'invention (décrite dans le tableau 13) ont été évaluées en déterminant l'efficacité de la composition à limiter les dépôts de microparticules de carbone (modèle de pollution atmosphérique). L'évaluation a été réalisée par analyse d'image sur photos standardisées réalisées à l'aide du vidéo-dermoscope C-Cube, par un essai clinique sur 10 volontaires selon le protocole suivant :
- Délimitation de 2 zones de 16 cm² (zone à traiter et zone témoin) au niveau des avantbras ;
- Application de 50 µL d'une dilution alcoolique de sébum synthétique à 15 % au niveau des zones prédéfinies ;
- Application de la composition Ex9.3 à raison de 2 mg/cm² sur la zone à traiter ;
- Dépôt de 50 µL d'une suspension hydro-alcoolique de particules de carbone à 1,2 % au niveau des zones prédéfinies ;
- Acquisition d'image (T0) à l'aide du vidéo-dermoscope C-Cube ;
- Essuyage standardisé des 2 zones à l'aide d'un carré ou d'un disque de coton humidifié, et séchage à l'air libre ;
- Acquisition d'image (T1) à l'aide du vidéo-dermoscope C-Cube.

Le tableau 15 ci-dessous rassemble le nombre de pixels sombres détectés avant essuyage (T0) et après essuyage (T1) de la zone témoin et de la zone à traiter. Le pourcentage de variation entre la zone témoin et la zone à traiter est également indiqué.

**[Tableau 15]**

| | T0 | | T1 | |
|---|---|---|---|---|
| | Zone traitée | Zone témoin | Zone traitée | Zone témoin |
| Moyenne (nombre de pixels sombres détectés) - Ex9.3 | 1426288 | 435687 | 1045398 | 4851 |
| Variation (%) - Ex9.3 | -69,45% | | -99,54% | |

Le pourcentage de variation montre que la composition cosmétique selon l'invention présente des propriétés anti-pollution puisqu'elle permet une diminution de plus de 99% des pixels représentatifs de particules de carbone, et donc de la pollution atmosphérique.

## Revendications

1. Composition épaississante pour une application topique sur la peau, les lèvres ou les phanères, ladite composition épaississante comprenant :
- Au moins une huile d'origine biologique, et
- Au moins un polymère poly(farnesène) ayant une masse molaire moyenne en nombre allant de 10000 à 120000 g/mol, ledit polymère poly(farnesène) étant partiellement ou totalement hydrogéné et/ou fonctionnalisé avec des hydroxyles.

2. Composition épaississante selon la revendication 1, dans laquelle l'huile d'origine biologique est choisie parmi les huiles hydrocarbonées, les huiles végétales ou animales sous forme d'acides, d'esters ou d'amides, et leurs mélanges.

3. Composition épaississante selon la revendication 1 ou 2, dans laquelle l'huile d'origine biologique est une huile hydrocarbonée, de préférence comprenant une teneur en poids d'isoparaffines allant de 90 à 100%, une teneur en poids de paraffines normales allant de 0 à 10% et une teneur en carbone d'origine biologique de préférence supérieure ou égale à 90 % par rapport au poids total de l'huile hydrocarbonée.

4. Composition épaississante selon la revendication 3, dans laquelle l'huile hydrocarbonée est choisie parmi les isoparaffines non-cycliques comprenant de 14 à 18 atomes de carbone.

5. Composition épaississante selon l'une quelconque des revendications 3 à 4, dans laquelle l'huile hydrocarbonée comprend :
- une teneur en poids d'isoparaffines allant de 95 à 100% et préférentiellement de 98% à 100% par rapport au poids total de l'huile hydrocarbonée ; et/ou
- une teneur en carbone d'origine biologique supérieure ou égale à 95%, de préférence supérieure ou égale à 98% et préférentiellement de 100% ; et/ou
- une teneur en poids de paraffines normales inférieure ou égale à 10, de préférence inférieure ou égale à 5% et préférentiellement inférieure ou égale à 2% par rapport au poids total de l'huile hydrocarbonée ; et/ou
- une teneur en poids de composés naphténiques inférieure ou égale à 1%, de préférence inférieure ou égale à 0,5% et préférentiellement inférieure ou égale à 100 ppm par rapport au poids total de l'huile hydrocarbonée ; et/ou
- une teneur en poids de composés aromatiques inférieure ou égale à 500 ppm, de préférence inférieure ou égale à 300 ppm, préférentiellement inférieure ou égale à 100 ppm, plus préférentiellement inférieure ou égale à 50 ppm et avantageusement inférieure ou égale à 20 ppm,
par rapport au poids total de l'huile hydrocarbonée.

6. Composition épaississante selon l'une quelconque des revendications 3 à 5, dans laquelle l'huile hydrocarbonée est obtenue par un procédé d'hydrogénation catalytique à une température de 80 à 180°C et à une pression de 50 à 160 bars d'une charge d'origine biologique désoxygénée et/ou isomérisée.

7. Composition épaississante selon l'une quelconque des revendications 1 à 6, dans laquelle le polymère poly(farnesène) présente une masse molaire moyenne en nombre allant de 20000 à 90000 g/mol, de préférence de 30000 à 85000 g/mol, de préférence encore de 40000 à 80000 g/mol, plus préférentiellement de 50000 à 80000 g/mol.

8. Composition épaississante selon l'une quelconque des revendications 1 à 7 comprenant, par rapport au poids total de la composition épaississante :
- de 10 à 90% en poids, de préférence de 20 à 80% en poids, de préférence encore de 30 à 70% en poids, encore plus préférentiellement de 40 à 60% en poids, d'huile(s) d'origine biologique, et
- de 10 à 90% en poids, de préférence de 20 à 80% en poids, de préférence encore de 30 à 70% en poids, encore plus préférentiellement de 40 à 60% en poids, de polymère(s) poly(farnesène).

9. Utilisation de la composition épaississante selon l'une quelconque des revendications 1 à 8, comme agent épaississant, agent sensoriel, agent filmogène, agent texturant, agent améliorant l'imperméabilité, agent hydratant, conditionneur, huile visqueuse ou agent ayant un effet seconde peau, dans une composition cosmétique comprenant de préférence au moins un corps gras.

10. Composition cosmétique pour une application cosmétique topique sur la peau, les lèvres ou les phanères, ladite composition cosmétique comprenant au moins une composition épaississante selon l'une des revendications 1 à 8, de préférence en une quantité allant de 1 à 80%, préférentiellement de 5 à 70% et avantageusement de 10 à 50% en poids par rapport au poids total de la composition cosmétique.

11. Composition cosmétique selon la revendication 10, comprenant au moins un corps gras choisi parmi : les huiles végétales le cas échéant autres que les huiles végétales de ladite composition épaississante, les huiles hydrocarbonées le cas échéant autres que l'huile hydrocarbonée de ladite composition épaississante, les beurres végétaux, les éthers et alcools gras, les esters huileux, les alcanes et les huiles silicones et/ou au moins un additif de préférence choisi parmi les émulsionnants.

12. Utilisation non-thérapeutique de la composition épaississante selon l'une quelconque des revendications
1 à 8, ou de la composition cosmétique selon la revendication 10 ou 11 pour une application topique, notamment comme produit de soin pour la peau ou des cheveux, comme produit de maquillage, comme produit capillaire, comme produit démaquillant, comme produit parfumé, comme produit de soin des lèvres, tel que le gloss ou les sticks hydratants pour les lèvres.

13. Procédé non-thérapeutique de traitement cosmétique de la peau, des lèvres ou des phanères comprenant
au moins une étape d'application, de préférence par étalement, de la composition épaississante selon l'une quelconque des revendications 1 à 8 ou de la composition cosmétique selon la revendication 10 ou 11, sur la peau, les lèvres ou les phanères.

14. Composition solaire pour une application topique, ladite composition solaire comprenant (i) au moins un filtre solaire et (ii) au moins une composition épaississante selon l'une des revendications 1 à 8, de préférence en une quantité allant de 1 à 80%, préférentiellement de 5 à 70% et avantageusement de 10 à 50% en poids par rapport au poids total de la composition solaire.

15. Composition épaississante selon l'une quelconque des revendications 1 à 8 ou composition solaire selon la revendication 14, pour une utilisation comme produit solaire.

## Patentansprüche

1. Verdickungszusammensetzung zur topischen Anwendung auf die Haut, die Lippen oder die Adnexen, wobei die Zusammensetzung umfasst:
- mindestens ein Öl biologischer Herkunft und
- mindestens ein Poly(farnesen)-Polymer mit einer zahlengemittelten Molmasse von 10000 - 120000 g/mol, wobei das Poly(farnesen)-Polymer teilweise oder ganz hydriert und/oder mit Hydroxylen funktionalisiert ist.

2. Verdickungszusammensetzung nach Anspruch 1, wobei das Öl biologischer Herkunft aus folgender Gruppe gewählt ist: Kohlenwasserstofföle, pflanzliche oder tierische Öle in Säure-, Ester- oder Amidform und Mischungen davon.

3. Verdickungszusammensetzung nach Anspruch 1 oder 2, wobei das Öl biologischer Herkunft ein Kohlenwasserstofföl ist, das, bezogen auf das Gesamtgewicht des Kohlenwasserstofföls, vorzugsweise einen Isoparaffingehalt von 90 - 100 Gew.-%, einen Gehalt an normalen Paraffinen von 0 - 10 Gew.-% und einen Gehalt an Kohlenstoff biologischer Herkunft vorzugsweise größer oder gleich 90 Gew.-% umfasst.

4. Verdickungszusammensetzung nach Anspruch 3, wobei das Kohlenwasserstofföl aus den 14 - 18 Kohlenstoffatome umfassenden nicht zyklischen Isoparaffinen gewählt ist.

5. Verdickungszusammensetzung nach einem der Ansprüche 3 - 4, wobei das Kohlenwasserstofföl, bezogen auf das Gesamtgewicht des Kohlenwasserstofföls, umfasst:
- einen Isoparaffingehalt von 95 - 100 Gew.-%, vorzugsweise 98 - 100 Gew.-%, bezogen auf das Gesamtgewicht des Kohlenwasserstofföls; und/oder
- einen Gehalt an Kohlenstoff biologischer Herkunft größer oder gleich 95 %, insbesondere größer oder gleich 98 % und vorzugsweise 100 %; und/oder
- einen Gehalt an normalen Paraffinen kleiner oder gleich 10 Gew.-%, insbesondere kleiner oder gleich 5 Gew-% und vorzugsweise kleiner oder gleich 2 Gew.-% bezogen auf das Gesamtgewicht des Kohlenwasserstofföls; und/oder
- einen Gehalt an Naphthenverbindungen kleiner oder gleich 1 Gew.-%, insbesondere kleiner oder gleich 0,5 Gew-% und vorzugsweise kleiner oder gleich 100 ppm bezogen auf das Gesamtgewicht des Kohlenwasserstofföls; und/oder
- einen gewichtsbezogenen Aromatengehalt kleiner oder gleich 500 ppm, insbesondere kleiner oder gleich 300 ppm, vorzugsweise kleiner oder gleich 100 ppm, besonders bevorzugt kleiner oder gleich 50 ppm und vorteilhafterweise kleiner oder gleich 20 ppm.

6. Verdickungszusammensetzung nach einem der Ansprüche 3 - 5, wobei das Kohlenwasserstoff durch ein katalytisches Hydrierungsverfahren bei einer Temperatur von 80 - 180 °C und einem Druck von 50 - 160 bar an einem deoxydierten und/oder isomerisierten biologischen Stoff erzeugt wird.

7. Verdickungszusammensetzung nach einem der Ansprüche 1 - 6, wobei das Poly(farnesen)-Polymer eine zahlengemittelte Molmasse von 20000 - 90000 g/mol, insbesondere 30000 - 85000 g/mol, bevorzugt 40000 - 80000 g/mol, besonders bevorzugt 50000 ä 80000 g/mol aufweist.

8. Verdickungszusammensetzung nach einem der Ansprüche 1 - 7, umfassend, bezogen auf das Gesamtgewicht der Zusammensetzung:
- 10 - 90 Gew.-%, insbesondere 20 - 80 Gew.-%, vorzugsweise 30 - 70 Gew.-%, besonders bevorzugt 40 - 60 Gew.-% ÖI(e) biologischer Herkunft und
- 10 - 90 Gew.-%, insbesondere 20 - 80 Gew.-%, vorzugsweise 30 - 70 Gew.-%, besonders bevorzugt 40 - 60 Gew.-% ÖI(e) Poly(farnesen)-Polymer(e).

9. Verwendung der Zusammensetzung nach einem der Ansprüche 1 - 8 als Verdickungsmittel, sensorisches Mittel, Filmbildner, Strukturfestiger, Mittel zur Verbesserung der Undurchlässigkeit, Feuchthaltemittel, Konditioniermittel, visköses Öl oder Mittel mit Zweithaut-Effekt in einer kosmetischen Zusammensetzung, die vorzugsweise mindestens ein Fett umfasst.

10. Kosmetische Zusammensetzung zur topischen kosmetischen Anwendung auf die Haut, Lippen oder Adnexen, wobei die kosmetische Zusammensetzung, bezogen auf das Gesamtgewicht der kosmetischen Zusammensetzung, mindestens eine Verdickungszusammmensetzung nach einem der Ansprüche 1 - 8 in einer Menge von 1 - 80 Gew.-%, insbesondere 5 - 70 Gew.-% und vorzugsweise 10 - 80 Gew.-% umfasst.

11. Kosmetische Zusammensetzung nach Anspruch 10, umfassend mindestens ein aus folgender Gruppe gewähltes Fett: pflanzliche Öle, ggf. andere pflanzliche Öle als die pflanzlichen Öle der Verdickungszusammensetzung, Kohlenwasserstofföle, ggf. andere als das Kohlenwasserstofföl der Verdickungszusammensetzung, Pflanzenbutter, Fettether und -alkohole, Ölester, Alkane und Silikonöle und/oder mindestens ein vorzugsweise aus den Emulgatoren gewählter Zusatzstoff.

12. Nicht therapeutische Verwendung der Verdickungszusammensetzung nach einem der Ansprüche 1 - 8 oder der kosmetischen Zusammensetzung nach Anspruch 10 oder 11 zur topischen Anwendung, insbesondere als Haut- oder Haarpflegeprodukt, Schminkprodukt, Haarbehandlungsprodukt, Abschminkprodukt, Duftprodukt, Lippenpflegeprodukt wie z.B. Lipgloss oder Lippenpflegestifte.

13. Nicht therapeutisches Verfahren zur kosmetischen Behandlung der Haut, Lippen oder Adnexen, umfassend mindestens einen Schritt des Anwendens, vorzugsweise des Anpinselns, der Verdickungszusammensetzung nach einem der Ansprüche 1 - 8 oder der kosmetischen Zusammensetzung nach Anspruch 10 oder 11 auf die Haut, Lippen oder Adnexen.

14. Sonnenschutzzusammensetzung zur topischen Anwendung, wobei die Sonnenschutzzusammensetzung, bezogen auf das Gesamtgewicht der Sonnenschutzzusammensetzung, umfasst: (i) mindestens einen Sonnenfilter und (ii) mindestens eine Verdickungszusammmensetzung nach einem der Ansprüche 1 - 8 in einer Menge von 1 - 80 Gew.-%, insbesondere 5 - 70 Gew.-% und vorzugsweise 10 - 50 Gew.-% umfasst.

15. Verdickungszusammensetzung nach einem der Ansprüche 1 - 8 oder Sonnenschutzzusammensetzung nach Anspruch 14 zur Verwendung als Sonnenschutzprodukt.

## Claims

1. A thickening composition intended for topical application on the skin, lips or skin appendages, said thickening composition comprising:
- At least one oil derived from a biological source, and
- At least one poly(farnesene) polymer having a number average molar mass ranging from 10,000 to 120,000 g/mol, said poly(farnesene) polymer being partially or fully hydrogenated and/or functionalised with hydroxyl groups.

2. The thickening composition according to claim 1, wherein the oil derived from a biological source is selected from among hydrocarbon oils, plant or animal oils in the form of acids, esters or amides, and mixtures thereof.

3. The thickening composition according to claim 1 or 2, wherein the oil derived from a biological source is a hydrocarbon oil, preferably comprising a content by weight of isoparaffins ranging from 90 to 100%, a content by weight of normal paraffins ranging from 0 to 10%, and a content of carbon derived from biological sources preferably greater than or equal to 90% relative to the total weight of the hydrocarbon oil.

4. The thickening composition according to claim 3, wherein the hydrocarbon oil is selected from among non-cyclic isoparaffins comprising from 14 to 18 carbon atoms.

5. The thickening composition according to any one of claims 3 to 4, wherein the hydrocarbon oil comprises:
- a content by weight of isoparaffins, ranging from 95 to 100% and preferentially from 98% to 100% relative to the total weight of the hydrocarbon oil; and/or
- a content of carbon derived from biological sources that is greater than or equal to 95%, preferably greater than or equal to 98%, and preferentially 100%; and/or
- a content by weight of normal paraffins that is less than or equal to 10, preferably less than or equal to 5%, and preferentially less than or equal to 2% relative to the total weight of the hydrocarbon oil; and/or
- a content by weight of naphthenic compounds that is less than or equal to 1%, preferably less than or equal to 0.5%, and preferentially less than or equal to 100 ppm relative to the total weight of the hydrocarbon oil; and/or
- a content by weight of aromatic compounds that is less than or equal to 500 ppm, preferably less than or equal to 300 ppm, preferentially less than or equal to 100 ppm, more preferentially less than or equal to 50 ppm, and advantageously less than or equal to 20 ppm,
relative to the total weight of the hydrocarbon oil.

6. The thickening composition according to any one of claims 3 to 5, wherein the hydrocarbon oil is obtained by a method of catalytic hydrogenation at a temperature of 80 to 180°C, and at a pressure of 50 to 160 bars of a deoxygenated and/or isomerised biologically-sourced feedstock.

7. The thickening composition according to any one of claims 1 to 6, wherein the poly(farnesene) polymer has a number average molar mass ranging from 20,000 to 90,000 g/mol, preferably 30,000 to 85,000 g/mol, more preferably from 40,000 to 80,000 g/mol, more preferentially from 50,000 to 80,000 g/mol.

8. The thickening composition according to any one of claims 1 to 7, comprising, relative to the total weight of the thickening composition:
- from 10 to 90% by weight, preferably from 20 to 80% by weight, more preferably from 30 to 70% by weight, even more preferentially from 40 to 60% by weight, of oil(s) derived from biological sources; and
- from 10 to 90% by weight, preferably from 20 to 80% by weight, more preferably from 30 to 70% by weight, even more preferentially from 40 to 60% by weight, of poly(farnesene) polymer(s).

9. The use of a thickening composition according to any one of claims 1 to 8, as a thickening agent, sensory agent, film-forming agent, texturing agent, water resistance enhancing agent, moisturising/hydrating agent, conditioner, viscous oil, or agent that provides a second skin effect, in a cosmetic composition that preferably comprises at least one fatty substance.

10. A cosmetic composition for topical cosmetic application on the skin, lips or skin appendages, said cosmetic composition comprising at least one thickening composition according to one of claims 1 to 8, preferably in an amount ranging from 1 to 80%, preferentially from 5 to 70%, and advantageously from 10 to 50% by weight, relative to the total weight of the cosmetic composition.

11. The cosmetic composition according to claim 10, comprising at least one fatty substance selected from among: plant oils, where appropriate other than the plant oils of said thickening composition; hydrocarbon oils, where appropriate other than the hydrocarbon oil of said thickening composition; plant butters; fatty ethers and fatty alcohols; oily esters; alkanes and silicone oils; and/or at least one additive preferably selected from emulsifiers.

12. The non-therapeutic use of the thickening composition according to any one of claims 1 to 8, or of the cosmetic composition according to claim 10 or 11, for topical application, in particular as a care product for the skin or the hair, as a makeup product, as a hair product, as a makeup removal product, as a perfumed product, as a sunscreen product, as a lip care product, such as a lip gloss or moisturising sticks for the lips.

13. A non-therapeutic process for the cosmetic treatment of the skin, lips or skin appendages, comprising at least one step of applying, preferably by spreading, the thickening composition according to any one of claims 1 to 8 or the cosmetic composition according to claim 10 or 11, on the skin, the lips or skin appendages.

14. A sunscreen composition for topical application, said sunscreen composition comprising (i) at least one sunscreen filter, and (ii) at least one thickening composition according to one of claims 1 to 8, preferably in an amount ranging from 1 to 80%, preferentially from 5 to 70%, and advantageously from 10 to 50% by weight relative to the total weight of the sunscreen composition.

15. A thickening composition according to any one of claims 1 to 8 or a sunscreen composition according to claim 14 for use as a sunscreen product.
